# EUROPEAN PATENT APPLICATION

(11) **EP 3 978 020 A1**
(43) Date of publication of application: **06.04.2022**
(21) Application number: 20778483.6
(22) Date of filing: 26.03.2020
(51) Int. Cl.: A61K 45/06

(54) **SKIN COMPOSITION**

(30) Priority: 27.03.2019 JP 2019059616
(71) Applicant: National University Corporation Tokyo Medical and Dental University, Tokyo 113-8510 (JP)
(72) Inventor: NISHIMURA Emi, Tokyo 113-8510 (JP)
(74) Representative: Plasseraud IP
(86) International application number: PCT/JP2020/013856
(87) International publication number: WO 2020/196801

(57) **Abstract**

The present invention pertains to: the provision of a method for promoting skin wound healing and a composition to be used for promoting skin wound healing; the provision of a method useful for preventing or ameliorating ulcers and bedsores or a composition therefor; the provision of a method for preventing skin aging and a composition to be used for preventing skin aging; the provision of a composition useful for preventing or ameliorating skin damage caused by an anticancer agent; and, furthermore, the provision of a method for enhancing the regeneration ability of epidermal stem cells and a composition for enhancing the regeneration ability of epidermal stem cells. More specifically, use of a composition, said composition being characterized by comprising, as an active ingredient, a substance selected from the group consisting of a substance which induces or maintains the expression of COL17A1 in cells, a substance which inhibits the degradation of COL17A1 in cells, a substance which promotes the competitive amplification ability of epidermal stem cells, a substance which suppresses genomic stress or oxidative stress in cells and a substance which prevents DNA damage in cells, enables the promotion of skin wound healing, protection from an anticancer agent and prevention of skin aging. Thus, the regeneration ability of epidermal stem cells can be enhanced.

## Description

### Technical Field

The present invention relates to compositions having beneficial effects on the skin. Specifically, the present invention relates to compositions having the effect of promoting skin wound healing, inhibiting skin aging, and/or enhancing the regenerative ability of epidermal stem cells. Furthermore, the present invention relates more specifically to the areas of pharmaceutical compositions, cosmetic compositions, and beauty supplements.

### Background Art

Aging of animal organs/internal organs is a multi-step process that results in their structural and functional decline. Details of the exact fate of damaged/stressed cells are unknown, although cellular aging and/or stem cell depletion have been implicated as cellular dynamics. In general, little is known about the *in vivo* dynamics of the cells that make up tissues/organelles during aging. Previous studies of mammalian hair follicles (skin mini-organs) have demonstrated the central role of stem cell aging in the progression of hair follicle aging and the existence of a "stem cell-centric organ aging program" that causes aging-related hair follicle miniaturization leading to age-related hair thinning and graying (Non-patent Document 1, Patent Document 2). This raises a new fundamental question: whether large solid internal organs (organs) such as the skin have a unique aging program that governs the cellular dynamics of organ aging.

The phenomenon of cell competition has been reported mainly in Drosophila, where it eliminates inappropriate mutant cells that arise in epithelial tissues during embryogenesis and cancer development. Stem cells are known to engage in "neutral stem cell competition" in some epithelial tissues, characterized by stochastic loss and replacement of stem cells, but the truth of whether this is a completely random selection of cell fate or a random occurrence of cell competition triggered by differences between cells is not known. In the epidermis, symmetric cell division (SCD) and asymmetric cell division (ACD) of epidermal stem cells (also called keratinocyte (cornified cell) stem cells or epidermal basal cell) are balanced to maintain epidermal homeostasis. However, the specific involvement and role of cell competition in the physiological homeostasis and aging of the organs of mammalian animals and in the cellular dynamics of the stratified epithelium has been unclear.

With age, human skin exhibits skin atrophy (thinning), fragility, dryness, abnormal pigmentation, and delay of skin wound healing and irritation. In the process, the skin loses its epidermal and dermal thicknesses, epidermal projections, epidermal keratinocytes, and reserves of functional dermal fibroblasts. The fragility of aging human skin is partly attributed to the remodeling of the dermal-epidermal junction, especially the hemidesmosomal (HD) component (the structure that connects epithelial cells to the extracellular matrix of the basement membrane). Indeed, destabilization of HD components such as type XVII collagen (COL17A1/BP180/BPAG2/type 17 collagen) in aging human skin has been reported. In addition, loss of the COL17A1 gene causes generalized atrophic benign epidermolysis bullosa (GABEB), a rare form of junctional epidermolysis bullosa (JEB) characterized by relatively mild skin fragility, atrophy, abnormality of skin pigmentation (pleomorphic skin atrophy), and alopecia. Consistent with the phenotype, previous studies have shown that the Col17a1 gene deletion in mice causes hair follicle aging with loss of hair follicle cells (Non-patent Document 2, Patent Document 1). However, the relationship between the expression level of COL17A1 in epidermal stem cells and the occurrence of aging traits (skin atrophy, fragility, reduced barrier function, pigmentation abnormality, hair loss, etc.) that are generally seen in healthy individuals' skin is unclear. The role of COL17A1 expression in maintaining skin youth, aging, and regeneration (wound healing), the correlation between the level of COL17A1 expression and the competitive self-renewal ability of stem cells, and the fact that COL17A1-mediated stem cell competition maintains stem cell quality (youth) have not been elucidated.

### Citation List

### Patent Documents

Patent Document 1: Japanese Patent Application Kokai Publication No. (JP-A) 2009-161509 (unexamined, published Japanese patent application)
Patent Document 2: International Publication WO2017122668

### Non-patent Documents

Non-patent Document 1: Matsumura, H. et al. Hair follicle aging is driven by transepidermal elimination of stem cells via COL17A1 proteolysis. Science 351, aad4395 (2016).

Non-patent Document 2: Tanimura et al., Cell Stem Cell Vol. 8, February 2011, pp. 177-187.

### Summary of the Invention

### Problems to be Solved by the Invention

It is one of the objectives of the present invention to provide methods and compositions for preventing and improving skin ulcers, that is, promoting skin wound healing. It is also one of the objectives to provide methods and compositions for reducing or preventing skin damage associated with cancer treatment. It is also one of the objectives of the present invention to provide methods for inhibiting skin aging and compositions for use in inhibiting skin aging and compositions for inhibiting aging of hair follicles which are an appendage of the skin. It is also one of the objectives of the present invention to provide methods for enhancing the regenerative ability of epidermal stem cells, compositions for enhancing the regenerative ability of epidermal stem cells (which may also include hair follicle stem cells), and compositions and methods for controlling stem cell competition.

In addition, it is one of the objectives of the present invention to provide a method for screening substances that are effective in reducing or preventing skin damage associated with cancer treatment, promoting skin wound healing, suppressing skin aging, regulating cell competition, and/or improving the regenerative ability of epidermal stem cells.

### Means for Solving the Problems

To elucidate the mechanisms of aging in mammalian organs using mice with identical genetic and environmental backgrounds, the present inventors utilized the tail skin of C57BL6 inbred mice, which exhibit age-related skin atrophy, fragility, pigmentation abnormality, and impaired wound healing as seen in aging human skin. The present inventors discovered that epidermal cell competition dynamics linked to COL17A1-mediated epidermal stem cell proliferation maintains skin homeostasis, thereby regulating aging of the skin organ. The present inventors also performed an *in vivo* cell fate tracking analysis of epidermal stem cells from aging mice and found that the heterogeneous appearance of COL17A1^{high} and COL17A1^{low} clones in the epidermis promotes their mutual cell competition through COL 17A1-mediated SCD, leading to the elimination of COL17A1^{low}, and promotion of the linked proliferation of COL17A1^{high} epidermal stem cell clones. Furthermore, the present inventors have shown that epidermal stem cell competition dynamics regulates the aging of the skin organ itself, by mediating the epidermal aging processes that accompany reduction of the skin regenerative ability and decreases in the epidermal melanocytes and dermal mesenchymal cells. In addition, by forcing the COL17A1 expression in epidermal stem cells and maintaining the competitive self-renewal potential of epidermal stem cells, the present inventors successfully prevented skin aging and improved wound healing, demonstrating that COL17A1 regulates skin organ aging through epidermal stem cell competition and maintains heterogeneous cells.

Cell competition has been implicated in the elimination of inappropriate cells, but its role in mammalian organ aging has been largely unknown. The present inventors have shown that epidermal stem cells sense their cytocompatibility/stress through the stability of the HD component, type XVII collagen (COL17A1), to promote cell competition for skin homeostasis and aging. *In vivo* cell fate tracking, clonal analysis, and *in vitro* 3D modeling revealed that COL17A1^{high} epidermal stem cells proliferate clonally via COL17A1-dependent symmetric cell division, thereby eliminating COL17A1^{low/-}-depleted cells from the skin to maintain homeostasis. Their dominant expansion by competition eventually minimizes their own COL17A1 levels, reducing not only their self-renewal ability but also the regenerative ability of adjacent epidermal melanocytes and dermal fibroblasts, causing skin atrophy, pigmentation abnormalities and poor wound healing. Furthermore, enforced maintenance of the COL17A1 expression in the epidermis rescues the skin aging phenotype. These findings open up new avenues for therapeutic intervention. The present invention is based on these findings and specifically relates to the embodiments below.

### Embodiment 1

A composition for use in promoting skin wound healing, or preventing or improving a skin ulcer or a bedsore, comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

### Embodiment 2

A composition for use in inhibiting skin aging, comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

### Embodiment 3

A composition for enhancing the regenerative ability of epidermal stem cells, comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

### Embodiment 4

A composition for use in preventing or improving a skin disorder caused by an anticancer agent, comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

### Embodiment 5

A composition for use in anti-wrinkle treatment, comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

### Embodiment 6

The composition according to embodiment 5, wherein the anti-wrinkle treatment is due to at least one of the following: improvement of skin elasticity, improvement of stratum corneum function, improvement of skin moisturizing ability, and improvement of skin barrier function.

### Embodiment 7

A composition for use in anti-spot treatment, comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

### Embodiment 8

A composition for use in improving rough skin, comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

### Embodiment 9

The composition of any one of embodiments 1-8, wherein the substance that induces or maintains the expression of COL17A1 in a cell further maintains the self-renewal ability of epidermal stem cells.

### Embodiment 10

The composition of any one of embodiments 1-9, wherein the substance that induces or maintains the expression of COL17A1 in a cell is selected from the group consisting of an NADPH oxidase inhibitor, a COX inhibitor, an iNOS inhibitor, a ROCK inhibitor, and an estrogen-like substance.

### Embodiment 11

The composition according to embodiment 10, wherein the NADPH oxidase inhibitor is selected from the group consisting of apocynin, ebselen, diphenyleneiodonium (DPI), GKT137831, AEBSF, GK-136901, ML171, Coenzyme Q10 (CoQ10), VAS2870, and VAS3947.

### Embodiment 12

The composition according to embodiment 10, wherein the COX inhibitor is selected from the group consisting of: celecoxib, deracoxib, tolfenamic acid, niflumic acid, FR122047, LM-1685, SC-791, BTB02472, Nimesulide, SPB04674, curcumin, diclofenac, 4'-hydroxydiclofenac, DuP-697, ebselen, ETYA, flubiprofen, ibuprofen, indomethacin, meloxicam, NPPB, NS-398, pterostilbene, resveratrol, SC-560, SKF-86002, TXA (tranexamic acid), TXC (Cetyl Tranexamate HCl), Panax Ginseng extract, and sulphide sulindac.

### Embodiment 13

The composition according to embodiment 10, wherein the iNOS inhibitor is selected from the group consisting of 1400W, L-NIL, aminoguanidine, BYK190123, S-ethylisothiourea, S-methylisothiourea, S-aminoethylisothiourea, 2-iminopiperidine, butylamine, ONO-1714 ((1S,5S,6R,7R)-7-chloro-3-imino-5-methyl -2-azabicyclo[4.1.0]heptane hydrochloride), AMT hydrochloride (2-amino-5,6-dihydro-6 -methyl-4H-1,3-thiazine hydrochloride), AR-C102222, L-NG-nitroarginine, L-NG-nitroarginine, L-NG-monomethylarginine, L-nitroarginine methyl ester, L-NIO, dexamethasone, estrogen, astaxanthin, and apigenin.

### Embodiment 14

The composition according to embodiment 10, wherein the ROCK inhibitor is selected from the group consisting of Y-27632, Ripasudil (K-115), Thiazovivin, Fasudil (HA-1077), GSK429286A, RKI-1447, GSK269962, Netarsudil (AR-13324), Y-39983, ZINC00881524, KD025, Hydroxyfasudil (HA-1100), GSK180736A and AT13148.

### Embodiment 15

The composition according to embodiment 10, wherein the estrogen-like substance is selected from the group consisting of estrogen, ethinylestradiol, biochanin A, Glycine Soja (Soybean) extract, isoflavone, Belamcanda Chinensis Root Extract, and Pueraria Mirifica Root Extract.

### Embodiment 16

The composition of any one of embodiments 1-9, wherein the substance that induces or maintains the expression of COL17A1 in a cell is selected from the group consisting of apocynin, ebselen, celecoxib, apigenin, Y-27632, Ripasudil, 1400W, ethinylestradiol, biochanin A, Necrostatin 1, Apocynum Venetum Leaf/Stem Extract, Morus Alba Leaf Extract, G ymnema Sylvestre Leaf Extract, Camellia Sinensis Leaf Extract, Eriobotrya Japonica Leaf Extract, Silybum Marianum Extract, Kaempferia Parviflora Extract, Plantago Major Seed Extract, Coffea Arabica (Coffee) Seed Extract, Prunus Yedoensis Leaf Extract, Melia Azadirachta Leaf Extract, Citrus Nobilis (Tangerine) Peel Extract, Lavandula Angustifolia (Lavender) Leaf Extract, Sophora Angustifolia Root Extract, Oryza Sativa (Rice) Bran Extract, Rosa Canina Fruit Extract, Hamamelis Virginiana (Witch Hazel) Leaf Extract, Eucalyptus Globulus Leaf Extract, Crataegus Cuneata Fruit Extract, and Panax Ginseng Root Extract.

### Embodiment 17

The composition of any one of embodiments 1-9, wherein the substance that inhibits the degradation of COL17A1 in a cell is selected from the group consisting of a neutrophils elastase (ELANE) inhibitor and a matrix metalloproteinase (MMP) inhibitor.

### Embodiment 18

The composition according to embodiment 17, the ELANE inhibitor is selected from the group consisting of sivelestat sodium hydrate(Monosodium N-{2-[4-(2,2-dimethylpropanoyloxy)-phenylsulfonylamino]benzoyl}aminoacetate tetrahydrate), ONO-6818 (2-(5-Amino-6-oxo-2-phenylhydropyrimidinyl)-N-[2-(5-tert-butyl-1,3,4-oxadiazol-2-yl)-1-(methylethyl)-2-oxoethylacetamide), α1-antitrypsin (α1-AT), Depelestat (Depelestat), NEI-L1 (sodium trifluoride isopropyl oxopropyl aminocarbonyl pyrrolidine carbonyl methyl propyl aminocarbonyl benzoylaminoacetate), Perilla Ocymoides Leaf Ferment Filtrate, Carum Petroselinum Ferment Filatrate, bell pepper ferment filtrate, antibodies against ELANE, siRNAs against a gene encoding ELANE, and antisense oligonucleotides against a gene encoding ELANE.

### Embodiment 19

The composition according to embodiment 17, wherein the MMP inhibitor is selected from the group consisting of Marimastat (Marimastat), Batimastat (Batimastat), PD166793, Ro32-3555, WAY170523, UK370106, TIMP1, TIMP2, TIMP3, and TIMP4.

### Embodiment 20

The composition of any one of embodiments 1-9, wherein the genomic stress is either stress of DNA damage due to ultraviolet light, radiation or an anticancer agent, or replication stress associated with cell division.

### Embodiment 21

The composition according to embodiment 2, wherein the skin aging is at least one selected from the group consisting of (a) to (f) below:
(a) thinning, weakening, atrophy, or loss of firmness of the epidermis, dermis, and/or adipose tissue;
(b) decrease of the epidermal barrier function or dryness of the skin;
(c) reduction or immaturation of hemidesmosomes in the basement membrane;
(d) loss of fibroblasts in the upper dermis, or crepe wrinkles or fine wrinkles due to dryness of the skin;
(e) wrinkles due to loss of collagen in the dermis; and
(f) spots, senile pigment freckles, or depigmented spots.

### Embodiment 22

The composition according to embodiment 2, for use in wrinkle inhibition.

### Embodiment 23

The composition of any one of embodiments 1-22, wherein the composition is a pharmaceutical composition.

### Embodiment 24

The composition of any one of embodiments 1-23, comprising a pharmaceutically acceptable excipient.

### Embodiment 25

The composition of any one of embodiments 1-24, wherein the composition is a liniment.

### Embodiment 26

The composition of any one of embodiments 1-22, wherein the composition is a cosmetic composition.

### Embodiment 27

The cosmetic composition according to embodiment 26, comprising the active ingredient at 0.01-15 % by weight.

### Embodiment 28

The cosmetic composition according to embodiment 26 or 27, wherein the composition is for use in skin care.

### Embodiment 29

The cosmetic composition of any one of embodiments 26-28, further comprising at least one of an anti-skin aging agent, a skin tone-adjusting agent, an anti-inflammatory agent, and a sunscreen agent.

### Embodiment 30

A method for screening a substance that is effective in promoting skin wound healing, or preventing or improving a skin ulcer, inhibiting skin aging, regulating cell competition, improving the regenerative ability of epidermal stem cells, preventing or improving a skin disorder caused by an anticancer agent, preventing wrinkles, and/or improving skin roughness, comprising:
(i) contacting cells with a test substance *in vitro*;
(ii) measuring the expression of COL17A1 in a cell; and
(iii) determining whether the test substance upregulates the expression of COL17A1.

### Embodiment 31

A method for assessing skin aging using the expression of COL17A1 in a cell as an indicator.

### Embodiment 32

A kit for use in a method for evaluating skin aging, comprising an antibody against COL17A1, a probe against a gene encoding COL17A1, or primers for amplifying the gene.

### Embodiment 33

A method for evaluating epidermal stem cells using the expression of COL17A1 in a cell as an indicator.

### Embodiment 34

A kit for use in a method for evaluating epidermal stem cells, comprising an antibody against COL17A1, a probe against a gene encoding COL17A1, or primers for amplifying the gene.

### Embodiment 35

A method for sorting epidermal stem cells using the expression of COL17A1 in a cell as an indicator.

### Embodiment 36

A kit for use in a method for sorting epidermal stem cells, including an antibody against COL17A1, a probe against a gene encoding COL17A1, or primers for amplifying the gene.

### Embodiment 37

A method for amplifying epidermal stem cells and/or epidermal cells, comprising measuring the expression level of COL17A1 using an antibody against COL17A1, a probe against a gene encoding COL17A1, or primers for amplifying the gene, and sorting epidermal stem cells with a high expression level of COL17A1.

### Embodiment 38

A kit for use in a method of amplifying epidermal stem cells and/or epidermal cells, comprising an antibody against COL17A1, a probe against a gene encoding COL17A1, or primers for amplifying the gene.

### Embodiment 39

A functional food or beauty supplement comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

### Embodiment 40

The functional food or beauty supplement according to embodiment 38, wherein the substance that induces or maintains the expression of COL17A1 in a cell is selected from the group consisting of an NADPH oxidase inhibitor, a COX inhibitor, an iNOS inhibitor, a ROCK inhibitor, and an estrogen-like substance.

### Embodiment 41

The functional food or beauty supplement according to embodiment 39 or 40, wherein the substance that induces or maintains the expression of COL17A1 in a cell is selected from the group consisting of apocynin, ebselen, celecoxib, apigenin, Y-27632, Ripasudil, 1400W, ethinylestradiol, biochanin A, Necrostatin 1, Apocynum Venetum Leaf/Stem Extract, Morus Alba Leaf Extract, Gymnema Sylvestre Leaf Extract, Camellia Sinensis Leaf Extract, Eriobotrya Japonica Leaf Extract, Silybum Marianum Extract, Kaempferia Parviflora Extract, Plantago Major Seed Extract, Coffea Arabica (Coffee) Seed Extract, Prunus Yedoensis Leaf Extract, Melia Azadirachta Leaf Extract, Citrus Nobilis (Tangerine) Peel Extract, Lavandula Angustifolia (Lavender) Leaf Extract, Sophora Angustifolia Root Extract, Oryza Sativa (Rice) Bran Extract, Rosa Canina Fruit Extract, Hamamelis Virginiana (Witch Hazel) Leaf Extract, Eucalyptus Globulus Leaf Extract, Crataegus Cuneata Fruit Extract, and Panax Ginseng Root Extract..

### Embodiment 42

The functional food or beauty supplement according to any one of embodiments 39-41, which is intended for oral intake.

### Embodiment 43

The functional food or beauty supplement according to any one of embodiments 39-42, which is in the form of tablet, powder, semi-solid, jelly or liquid.

### Embodiment 44

The functional food or beauty supplement according to any one of embodiments 39-43, further comprising at least one of an anti-skin aging agent, vitamin, collagen, and mineral.

### Embodiment 45

A cell composition comprising an isolated cell expressing COL17A1.

### Embodiment 46

The cell composition according to embodiment 45, wherein the cell is an epidermal keratinocyte or a mucosal epithelial keratinocyte.

### Embodiment 47

The cell composition according to embodiment 45 or 46, which is for use in transplantation.

### Embodiment 48

The cell composition according to any one of embodiments 45-47, which is for use in the treatment of a disease or injury of the skin.

### Embodiment 49

The cell composition according to any one of embodiments 45-48, comprising at least 1 × 10³ cells.

### Embodiment 50

The cell composition according to any one of embodiments 45-49, wherein at least 50% of the cells express COL17A1.

### Embodiment 51

The cell composition according to any one of embodiments 45-50, wherein the cell is a stem cell-derived cell.

### Embodiment 52

The cell composition according to any one of embodiments 45-51, wherein the cell is frozen.

### Embodiment 53

The cell composition according to any one of embodiments 45-52, wherein the cell is contained in a single container.

### Embodiment 54

The cell composition according to any one of embodiments 45-53, wherein the cell is a cell treated with a substance that induces or maintains the expression of COL17A1 in the cell.

### Embodiment 55

The cell composition according to embodiment 53, wherein the substance that induces or maintains the expression of COL17A1 in the cell is selected from the group consisting of an NADPH oxidase inhibitor, a COX inhibitor, an iNOS inhibitor, a ROCK inhibitor, and an estrogen-like substance.

### Embodiment 56

The cell composition according to embodiment 54 or 55, wherein the substance that induces or maintains the expression of COL17A1 in a cell is selected from the group consisting of apocynin, ebselen, celecoxib, apigenin, Y-27632, Ripasudil, 1400W, ethinylestradiol, biochanin A, Necrostatin 1, Apocynum Venetum Leaf/Stem Extract, Morus Alba Leaf Extract, Gymnema Sylvestre Leaf Extract, Camellia Sinensis Leaf Extract, Eriobotrya Japonica Leaf Extract, Silybum Marianum Extract, Kaempferia Parviflora Extract, Plantago Major Seed Extract, Coffea Arabica (Coffee) Seed Extract, Prunus Yedoensis Leaf Extract, Melia Azadirachta Leaf Extract, Citrus Nobilis (Tangerine) Peel Extract, Lavandula Angustifolia (Lavender) Leaf Extract, Sophora Angustifolia Root Extract, Oryza Sativa (Rice) Bran Extract, Rosa Canina Fruit Extract, Hamamelis Virginiana (Witch Hazel) Leaf Extract, Eucalyptus Globulus Leaf Extract, Crataegus Cuneata Fruit Extract, and Panax Ginseng Root Extract.

### Embodiment 57

A cell sheet comprising the cell composition according to any one of embodiments 45-56.

### Embodiment 58

A composition for use in regulating cell competition, comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

### Embodiment 59

A method for assessing cell competition in keratinocytes by culturing keratinocytes in three dimensions.

### Embodiment 60

A kit for use in a method for assessing cell competition in keratinocytes, comprising keratinocytes and a three-dimensional culture device.

### Brief Description of the Drawings

[Figure 1] Figure 1 shows graphs quantifying the fluorescence intensity of HD and basement membrane components, COL17A1, ITGA6, PLECTIN, ITGB4, COL7A1 and ITGB1 in the tail of young (7-8 weeks, n = 50) and old (22-25 months, n = 50) mice. The expressions of COL17A1 and PLECTIN were significantly decreased in aged skin.
[Figure 2] Figure 2 is a graph quantifying the number of HDs in the basement membrane in caudal basal cells from young (8 weeks, n = 3) and old (22 months, n = 3) mice. Physiological aging significantly reduced the number of HDs in the caudal basal cells.
[Figure 3] Figure 3 shows graphs quantifying the area (µm²) (left) and the number (right) of monochromatic clones in whole-mount images of tail epidermis during aging (4.5 months old, n = 5 images; 15 months old, n = 4 images; 24 months old, n = 7 images).
[Figure 4] Figure 4 shows immunostaining images of COL17A1 in multicolor labeled caudal basal keratinocytes derived from young (11 weeks old) and old (28 months old) Tamoxifen (TAM)-treated Epi-Confetti mice.
[Figure 5] Figure 5 is a graph quantifying the distribution between COL17A1 intensity and keratinocyte clone size in multiple regions of Figure 4 (11 weeks old, n = 13 clones; 28 months old, n = 12 clones).
[Figure 6] Figure 6 is a graph quantifying the floating clones in control (Cont) mice (D2, n = 4; D28, n = 5) and Col17a1 cKO mice (D2, n = 3; D28, n = 5) at day 2 (D2) and day 28 (D28) after TAM administration.
[Figure 7] Figure 7 shows graphs analyzing the colony formation of tail epidermal keratinocytes in young (7 weeks old, n = 3) and old (25 months old, n = 3) mice. The number and size of colonies were significantly decreased with age.
[Figure 8] Figure 8 shows graphs analyzing the colony formation of tail epidermal keratinocytes from wild-type (8 weeks old, n = 3) and hCOL17A1 tg (8 weeks old, n = 3) mice. The expression of hCOL17A1 tg significantly increased the number and size of colonies in the primary tail epidermal keratinocytes.
[Figure 9] Figure 9 presents graphs showing changes of pigment cells with aging. Loss of pigment cells in the epidermis due to aging caused pigmentation abnormalities in the skin (mixed deposition and loss) (left, center), but forced maintenance of COL17A1 maintained pigment cells and suppressed pigmentation abnormalities (right).
[Figure 10] Figure 10 is a graph showing the number of PDGFRa+ cells in the wild type and hCOL17A1tg mice. The decrease in PDGFRa+ cells directly below the basement membrane (BM) with aging (left) and their suppression by forced maintenance of COL17A1 (right) are shown.
[Figure 11] Figure 11 shows graphs quantifying the unrepaired wound area at day 0 (D0), day 14 (D14) and day 21 (D21). Wound repair was significantly delayed by physiological aging (left), or deficiency of Col17a1 or Itga6 (right).
[Figure 12] Figure 12 shows graphs quantifying the unrepaired wound area at day 0 (D0), day 14 (D14) and day 21 (D21). Wound repair was significantly accelerated by the expression of hCOL17A1 (left), or treatment with apocynin or Y-27632 (right).
[Figure 13] Figure 13 presents graphs showing the results of colony formation assay. The number of colonies (n = 3 wells) was significantly increased by Y-27632 treatment compared to the control (Cont) (left). The number of colonies (n = 3 wells) with a diameter greater than 2 mm was also significantly increased by Y-27632 or apocynin treatment (right).
[Figure 14] Figure 14 is a graph showing the results of colony formation assay. The average size of the top five colonies (n = 3 wells) was significantly increased by treatment with Y-27632 or apocynin compared to the control (Cont).
[Figure 15] Figure 15 shows graphs of HCS analysis of the COL17A1 expression (corresponding to Step 1 of the screening for COL17A1-mediated competitive self-renewal enhancers). Treatment with apocynin or ebselen significantly increased the amount of COL17A1 compared to the control in HCS.
[Figure 16] Figure 16 shows photographs of the colony formation assay (corresponding to Step 2 of the screening for COL17A1 expression-mediated competitive self-renewal enhancers) and graphs of analyzing the results. Treatment with apocynin or ebselen significantly increased the number and area of colonies per well compared to the control.

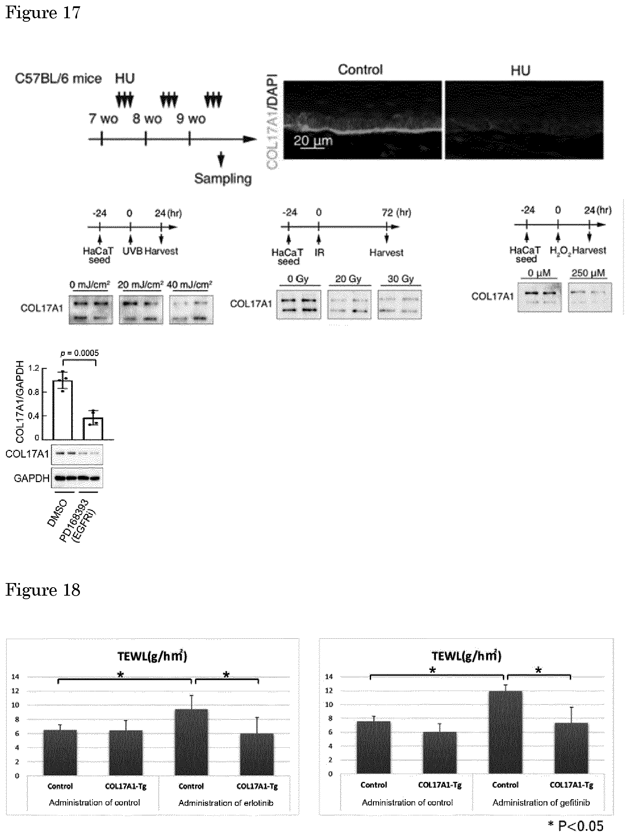

[Figure 17] Figure 17 shows decreased expression of COL17A1 by an anticancer drug and various stresses. The upper panel shows an immunostaining image of COL17A1 expression in the skin of C57BL/6 mice 3 months after continuous administration of hydroxyurea (HU). The middle left panel shows Western blot images of the COL17A1 level in HaCaT cells assessed 24 hours after UV irradiation. The center panel shows Western blot images of the COL17A1 level in HaCaT cells assessed 72 hours after irradiation. The middle right panel shows Western blot images of the COL17A1 level in HaCaT cells assessed 24 hours after hydrogen peroxide treatment. The lower panel shows a Western blot image of the COL17A1 level in HaCaT cells treated with an EGF receptor inhibitor (PD 168393).
[Figure 18] Figure 18 presents graphs of showing the effect of ameliorating skin lesions caused by an anticancer drug by over-expressing COL17A1.
[Figure 19] Figure 19 presents graphs of showing the effect of a competitive amplifier of epidermal stem cells in ameliorating skin lesions caused by administering an anticancer drug. The left panel is a graph showing TEWL on day 12 after continuous administration of an EGF receptor inhibitor (erlotinib) to 7-week-old C57BL/6 mice. The right panel is a graph showing TEWL on day 7 after continuous administration of an EGF receptor inhibitor (erlotinib) to 6-month-old C57BL/6 mice.
[Figure 20] Figure 20 presents images showing the preventive and therapeutic effects of apocynin in a model of skin lesions (dry eczema and alopecia) induced in the upper cervical-dorsal skin of aged mice.
[Figure 21] Figure 21 presents bar graphs showing the effect of substances that promote the cell competitive ability of epidermal stem cells in maintaining the stratum corneum water content (upper) and inhibiting the increase of TEWL (lower) in response to UV irradiation.
[Figure 22] Figure 22 presents bar graphs showing the effects of a competitive amplifier of epidermal stem cells in inhibiting the increase of TEWL (upper) and improving the loss of skin elasticity (lower) caused by UV irradiation in epidermal stem cells.

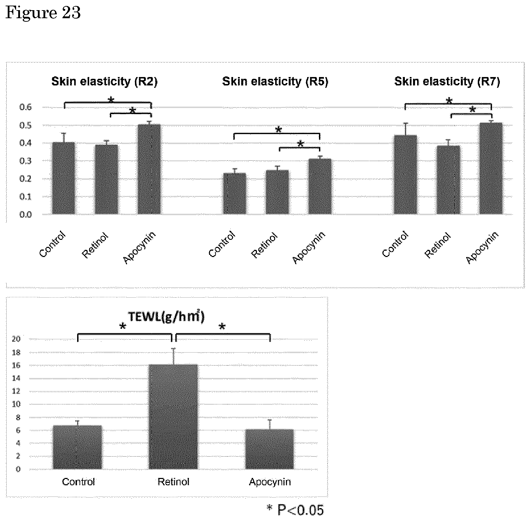

[Figure 23] Fig. 23 presents graphs showing the effect of the competitive amplifier of epidermal stem cells and the comparative agent on skin elasticity after long-term continuous application in epidermal stem cells (upper), and a bar graph showing the TEWL of epidermal stem cells after long-term continuous application of the competitive amplifier of epidermal stem cells and the comparative agent (lower). Retinol, known as a wrinkle reducer, significantly increased TEWL upon long-term application. On the other hand, the TEWL value of the substance that promotes the cell competitive ability of epidermal stem cells is not much different from that of the control, suggesting that it has the effect of improving skin elasticity while maintaining the barrier function of the skin.
[Figure 24] Figure 24 presents high-resolution photographic images and colored images of wrinkles (upper) showing the effects of a competitive amplifier of epidermal stem cells and a comparative agent in improving wrinkles caused by UV irradiation in epidermal stem cells, as well as bar graphs showing total wrinkle volume (mm³) and total wrinkle average depth (µm).
[Figure 25] Figure 25 is a bar graph showing the TEWL of epidermal stem cells after long-term application of a competitive amplifier of epidermal stem cells. Retinol, which is known as a wrinkle remedy, significantly increased TEWL upon long-term application. On the other hand, the TEWL value of the substance that promotes the cell competitive ability of epidermal stem cells was not much different from that of the control, suggesting that it has the effect of improving wrinkles while maintaining the barrier function of the skin.
[Figure 26] Figure 26 is a bar graph showing the effect of a substance that promotes the cell competitive ability of epidermal stem cells and a comparative agent on the reduction of skin flexibility caused by UV irradiation.
[Figure 27] Figure 27 presents high-resolution photographic images and colored images of texture showing improvement of the skin roughness induced by SDS with a competitive amplifier of epidermal stem cells (apocynin). The graphs show Ra (arithmetic mean roughness) and Rz (ten-point average roughness), which are indicators of skin surface roughness.
[Figure 28] Figure 28 is a bar graph showing the inhibitory effect of a substance (apocynin) that promotes the cell competitive ability of epidermal stem cells on the enhancement of TEWL induced by SDS.
[Figure 29] Figure 29 presents high-resolution photographic images and colored images of texture showing improvement of the skin roughness induced by SDS with a competitive amplifier of epidermal stem cells (ebselen). The graphs show Ra (arithmetic mean roughness) and Rz (ten-point average roughness), which are indicators of skin surface roughness.
[Figure 30] Figure 30 is a bar graph showing the inhibitory effect of a substance (apocynin) that promotes the cell competitive ability of epidermal stem cells on the enhancement of TEWL induced by SDS.
[Figure 31] Figure 31 presents photographs showing the effects of competitive amplifiers of epidermal stem cells (apocynin, ebselen, and celecoxib) on SDSinduced primary irritant skin damage (skin roughness) and pigmentation. On day 16 after the start of SDS treatment, the site of apocynin application was healed, while the control showed hyperpigmentation.
[Figure 32] Figure 32 presents high-resolution photographic images and colored images of texture showing the improvement of human skin roughness induced by SDS with competitive amplifiers of epidermal stem cells (apocynin, ebselen, and celecoxib). The graphs show "ΔRz" and "ΔRy" as the values subtracted from the control on measurement day for "Ry (maximum height)" and "Rz (ten-point average roughness)", which are indices of skin surface roughness.
[Figure 33] Figure 33 is a graph showing the effect of competitive amplifiers of epidermal stem cells (apocynin, ebselen, and celecoxib) on inhibiting the rise of TEWL associated with human skin roughness induced by SDS. Error bars in the graph indicate S. D. (standard deviation).

### Mode for Carrying Out the Invention

As described above, the present inventors found that COL17A1 (type XVII collagen) is involved in skin aging and regeneration, and identified a composition useful for promoting skin wound healing, inhibiting skin aging, regulating cell competition, and improving the regenerative ability of epidermal stem cells. The present invention is described in detail below.

### Skin wound healing

Skin wounds occur for a variety of reasons, such as trauma, cuts, burns, poor circulation, ulcers from bedsores, diabetes, etc., and temporarily impair normal function and structure of the skin. If the body is unable to heal these wounds, they may become chronic and eventually become infected. Skin wounds can be classified into acute skin wounds and chronic skin wounds. Acute skin wounds are wounds in which the wound healing mechanism works normally, such as fresh trauma and surgical wounds, while chronic skin wounds are wounds that have some causes that prevents the normal wound healing mechanism from working. Reasons that delay the healing of chronic skin wounds can be divided into two major categories: systemic factors such as underlying diseases, and local factors. One percent of patients will develop chronic skin wounds, but half of these wounds will never heal.

The skin is the largest organ in the human body and acts as a natural barrier to the outside world, protecting the interior tissues from wear and infection. The skin is generally classified into three layers: epidermis, dermis, and subcutaneous tissue. The epidermis is the outermost layer, and contains keratinocytes (cornified cells) as well as melanocytes (pigment cells). Keratinocytes form the epidermis, and they are lined in multiple layers and cover the outer surface of the body. Melanocytes are distributed in the basal layer of the epidermis and provide melanin pigment to the surrounding keratinocytes. The distribution of pigment cells, the amount of melanin pigment, and the type of melanin determine the color tone of the skin.

The epidermis is further classified into four layers from depth: stratum basale (basal cell layer), stratum spinosum (spinous cell layer), stratum granulosum (granular cell layer), and stratum corneum (horny cell layer). The stratum basale consists of one layer of basal cells containing the stem cells of keratinocytes. The stratum basale has desmosomes, gap junctions (gap junctions), and hemidesmosomes as structures for binding to adjacent cells and the basement membrane beneath the basal cells. The spinous layer consists of five to ten layers. This layer is called spinous cells because the cells appear to be connected to each other by thorns. The granulosum consists of two to three layers, and it is called by this term because it contains keratohyaline granules, which are basophilic and enriched in profilaggrin. The stratum corneum, also called the horny layer, consists of about 10 layers. Denucleated and dead keratinocytes become membranous and are stratified like fallen leaves. In the palms and soles, the stratum corneum is extremely thick, with the clear layer (stratum lucidum) directly below it. The cytoplasm of the cornified cells is filled with aggregated keratin fibers. In addition, hair follicles and sweat glands are present in continuity with the epidermis, and grow hair as skin appendages and discharge sweat.

Epidermal stem cells, which reside in the basal layer of the epidermis, become the driving force behind epidermal regeneration. They divide horizontally in relation to the basement membrane in a balanced manner through equal division or unequal division through vertical division. They then maintain the construction of the epidermis by supplying differentiated epidermal keratinocytes toward the skin surface through self-renewal. Keratinocytes make up the majority of cells in the epidermis. The cells found in the stratum corneum on the surface of the skin are almost all dead cells, and as these dead cells are shed, they are replaced by new keratinocytes that rise from the lower layers. This process of lifting from below is constantly repeated, and the entire skin surface is renewed in 15 to 30 days.

Beneath the epidermal layer is the basement membrane, which separates it from the underlying dermal layer. The dermal layer consists of fibroblasts, blood vessels, immune cells, skin appendages (hair follicles, sweat glands), and the extracellular matrix that supports these cells. Blood vessels nourish the skin, immune cells protect the skin, and fibroblasts produce collagen proteins to give it strength, as well as elastin protein to give it elasticity.

At the deepest part of the skin is the subcutaneous tissue. The subcutaneous tissue is a layer of fat beneath the dermis layer and functions as another barrier for the underlying muscles and bones, absorbing shock and protecting against infection.

Wounds form when the epidermal layer of the skin is broken down due to trauma, infection, anticancer drugs, age-related skin atrophy, breakdown of the barrier function, or other skin diseases, and the severity of the wound increases as the underlying layers of the skin become compromised. Three steps are required for a wound to heal normally: inflammation, proliferation, and remodeling. Inflammation lasts for approximately four days. The main event that occurs during this period is the creation of a fibrin matrix and the formation of blood clots to cover and protect the wound. During the proliferative phase, the inflammatory signal attracts many types of cells to the wound. The cells that are recruited include fibroblasts and vascular endothelial cells. Fibroblasts produce collagen and can also transform into myofibroblasts, which can close the wound. Vascular endothelial cells create new blood vessels around the wound. These cellular activities lead to the formation of a tissue called the "granulation tissue" immediately below the blood clot. The granulation tissue refers to the new tissue that is created as a repair and inflammatory response to tissue damage, and is composed of new blood vessels, connective tissue, fibroblasts, and inflammatory cells. Next, epidermal regeneration occurs from the epidermis surrounding the site of lesion and skin appendages, and keratinocytes migrate from the periphery of the wound to the wound and also from the base of the hair follicle. For defects in the deep skin with no residual appendages, the epidermis extends from the periphery after the wound surface has been replaced by granulation tissue. On top of the granulation tissue, keratinocytes go through cell division, bringing the epidermis together. During the remodeling phase, this process occurs continuously, the epidermis recovers as epidermal keratinocytes migrate from the periphery of the wound or the residual appendages, keratinocytes and fibroblasts produce matrix proteins for the newly formed basement membrane, and the interface between the epidermis and dermis is regenerated.

Hair follicles and sweat glands may also be destroyed when damage occurs deep in the dermis and into the adipose tissue, such as in the case of severe deep burns or cuts, reducing the number of keratinocytes available for epidermal repair. As a result, full thickness wounds take longer to heal and may require skin grafting (transplantation), treatment with cultured skin such as epidermal sheets, or transplantation with epidermal keratinocyte sprays.

### Composition for use in promoting skin wound healing, or preventing or improving skin ulcers or bedsores

One of the embodiments of the present invention relates to a composition for use in promoting skin wound healing or preventing or improving skin ulcers or bedsores, comprising as an active ingredient a substance that induces or maintains the expression of COL17A1 in a cell, a substance that inhibits the degradation of COL17A1 in a cell, a substance that inhibits genomic stress or oxidative stress in a cell, a substance that inhibits DNA damage (DNA damage response or DNA damage itself) in a cell (epidermal stem cells).

### Substances that induce or maintain the expression of COL17A1 in a cell

As described above, the present inventors succeeded in preventing skin aging and improving or promoting wound healing, or preventing or improving skin ulcers or bedsores by forcibly maintaining the expression of COL17A1 in epidermal stem cells, and showed that COL17A1 regulates skin organ aging through epidermal stem cell competition and heterogeneous cell maintenance. The present inventors have also shown that the matrix metalloproteinase (MMP) inhibitor, Marimastat, stabilizes COL17A1 and blocks the UV-induced downward repression of COL17A1. The present inventors have also identified a ROCK inhibitor (Y27632) and an NADPH oxidase inhibitor (apocynin) as chemicals that maintain or induce the COL17A1 expression in keratinocytes *in vitro* and promote maintenance of the self-renewal ability in cultured keratinocytes.

Furthermore, the present inventors have shown that similar to transgenic mice overexpressing human COL17A1, the two pharmaceutical agents Y27632 (a ROCK inhibitor) and apocynin (an NADPH oxidase inhibitor) significantly promote the wound repair process both *in vitro* and *in vivo.* Thus, it is understood by those skilled in the art that substances that induce or maintain the expression of COL17A1 in a cell or inhibit the degradation of COL17A1 in a cell can be used to promote or improve skin wound healing, or prevent or improve skin ulcers or bedsores. Furthermore, it is understood that substances that maintain the self-renewal ability of epidermal stem cells, substances that inhibit genomic stress or oxidative stress in a cell, and substances that inhibit DNA damage in a cell can be used to promote or improve skin wound healing or prevent or improve skin ulcers or bedsores. It is understood that substances that inhibit genomic stress or oxidative stress in a cell and substances that inhibit DNA damage in a cell can be used to promote or improve skin wound healing or prevent or improve skin ulcers or bedsores.

COL17A1 (type XVII collagen/BP180/BPAG2) is a transmembrane protein-type cell adhesion molecule and a protein of hemidesmosomes (half desmosomes) which are one of the cell junctions in epithelial tissues. Substances that induce or maintain the expression of COL17A1 in a cell include, but are not limited to, ROCK inhibitors, NADPH oxidase inhibitors, iNOS inhibitors, and COX inhibitors. Substances that induce or maintain the expression of COL17A1 in a cell also include DNAs or RNAs encoding COL17A1, which may be introduced into the cells using an appropriate vector.

ROCK inhibitors include, but are not limited to, Y27632, Thiazovivin (thiazovivin), Fasudil (HA-1077), GSK429286A, RKI-1447, GSK269962, Netarsudil (AR-13324), Y-39983, ZINC00881524, KD025, Ripasudil (K-115), Hydroxyfasudil (HA-1100), GSK180736A, and AT13148. NADPH oxidase inhibitors include, for example, apocynin, ebselen, diphenyleneiodonium (DPI), GKT137831, AEBSF, GK-136901, ML171, Coenzyme Q10 (CoQ10), VAS2870, VAS3947, but are not limited thereto. Plant extracts such as the Apocynum Venetum Leaf/Stem Extract containing apocynin are also included.

Among the plant extracts in the present invention, Apocynum Venetum Leaf/Stem Extract (trade name: Venetron), Morus Alba Leaf Extract (trade name: Morus Alba leaf extract powder), Gymnema Sylvestre Leaf Extract (trade name: Gymnema Sylvestre extract powder), Camellia Sinensis Leaf Extract (trade name: Tiacalon 90), Eriobotrya Japonica Leaf Extract (trade name: Eriobotrya Japonica Leaf powder), Silybum Marianum Extract (trade name: Silybum Marianum extract powder), and Silybum Marianum Extract (trade name: SIRTMAX) are manufactured by Tokiwa Phytochemical Co. Ltd., and are available. Plantago Major Seed Extract (trade name: Absorage), Coffea Arabica (Coffee) Seed Extract (trade name: Caffenoage), Prunus Yedoensis Leaf Extract (trade name: Sakura extract B), Melia Azadirachta Leaf Extract (trade name: Neem leaf liquid B), Citrus Nobilis (Tangerine) Peel Extract (trade name: MandarinClear), Lavandula Angustifolia (Lavender) Leaf Extract (trade name: ecofarm Lavender B), Sophora Angustifolia Root Extract(trade name: Pharcolex Clara B), Oryza Sativa (Rice) Bran Extract(trade name: Pharcolex rice nuka BK), Rosa Canina Fruit Extract(trade name: Pharcolex Novara B), Hamamelis Virginiana (Witch Hazel) Leaf Extract (trade name: Pharcolex Hamameris B), Eucalyptus Globulus Leaf Extract (trade name: Pharcolex Eucalyptus B), and Crataegus Cuneata Fruit Extract (trade name: Pharcolex Hawthorn B) are listed in the product guide of Ichimaru Pharcos Co., Ltd., and are easily available.

For the iNOS inhibitior, both selective iNOS inhibitors and nonselective iNOS inhibitors can be used, but selective iNOS inhibitors are preferred, for example, in terms of side effects.

The iNOS inhibitor includes, but is not limited to, for example, 1400W, L-NIL, aminoguanidine, BYK190123, S-ethyl isothiourea, S-methyl isothiourea, S-aminoethyl isothiourea, 2-imino-piperidine, butylamine, ONO-1714 (fused piperidine derivative; (1S,5S,6R,7R)-7-chloro-3-imino -5-methyl-2-azabicyclo[4.1.0]heptane hydrochloride), AMT hydrochloride (2-amino-5,6 - dihydro-6-methyl-4H-1,3-thiazine hydrochloride), AR-C102222, L-NG-nitroarginine, L-NG-monomethylarginine, L-nitroarginine methyl ester, L-NIO, dexamethasone, estrogen, and astaxanthin, as well as apigenin, which is an inhibitor of the iNOS expression.

The COX inhibitor includes, for example, celecoxib, deracoxib, tolfenamic acid, FR122047, LM-1685, SC-791, BTB02472, Nimesulide, SPB04674, curcumin, diclofenac, 4'-hydroxydiclofenac, DuP-697, ebselen, ETYA, flubiprofen, ibuprofen, indomethacin, Meloxicam, niflumic acid, NPPB, NS-398, pterostilbene, resveratrol, SC-560, SKF-86002, TXA (tranexamic acid), and TXC (Cetyl Tranexamate HCl). For the COX inhibitor, both selective COX inhibitors and nonselective COX inhibitors can be used, but selective COX-2 inhibitors are preferred, for example, in terms of side effects.

Estrogen-like substances include, for example, estrogen, ethinylestradiol, biochanin A, Glycine Soja (Soybean) Extract, isoflavone, Belamcanda Chinensis Root Extract, and Pueraria Mirifica Root Extract, but are not limited thereto.

### Substances that inhibit the degradation of COL17A1 in a cell

Substances that inhibit the degradation of COL17A1 in a cell include, for example, neutrophil elastase (ELANE) inhibitors and MMP inhibitors. The ELANE inhibitor includes, for example, sivelestat sodium hydrate (Monosodium N-{2-[4-(2,2-dimethylpropanoyloxy)-phenylsulfonylamino]benzoyl}aminoacetate tetrahydrate; also called elaspol), ONO-6818 (2-(5-Amino-6-oxo-2-phenylhydropyrimidinyl)-N-[2-(5-tert-butyl-1,3,4-oxadiazol-2-yl)-1 methylethyl)-2-oxoethyl]acetamide), α1-antitrypsin (α1-AT; A1AT), Depelestat (also called EPI-hNE4 or DX-890), NEI-L1 (sodium trifluoride isopropyl oxopropyl aminocarbonyl pyrrolidine carbonyl methyl propyl aminocarbonyl benzoylaminoacetate), fermented shiso leaves (JP 2006-61091), fermented parsley (JP 2006-75085), and fermented sweet pepper (JP 2006-76926), but are not limited thereto.

The ELANE inhibitor may be an antibody against ELANE, an siRNA against the gene encoding ELANE, an antisense oligonucleotide against the gene encoding ELANE, or such. The MMP inhibitor includes, for example, Marimastat, Batimastat, PD166793, Ro32-3555, WAY170523, UK370106, TIMP1, TIMP2, TIMP3, TIMP4, and such, but is not limited thereto.

### Substances that maintain the self-renewal ability of epidermal stem cells

The present inventors have discovered that the maintenance of epidermal stem cells is enabled by the expression and maintenance of COL17A1 and is based on the self-renewal ability of epidermal stem cells. Therefore, the substances that maintain the self-renewal ability of epidermal stem cells in the present invention include ROCK inhibitors, NADPH oxidase inhibitors, iNOS inhibitors, and COX inhibitors, as well as growth factors and plant extracts. Examples include apocynin, ebselen, celecoxib, apigenin, Y-27632, Ripasudil, 1400W, astaxanthin, ethinylestradiol, biochanin A, Necrostatin 1, Apocynum Venetum Leaf/Stem Extract, Morus Alba Leaf Extract, Gymnema Sylvestre Leaf Extract, Camellia Sinensis Leaf Extract, Eriobotrya Japonica Leaf Extract, Silybum Marianum Extract, Kaempferia Parviflora Extract, Plantago Major Seed Extract, Coffea Arabica (Coffee) Seed Extract, Prunus Yedoensis Leaf Extract, Melia Azadirachta Leaf Extract, Citrus Nobilis (Tangerine) Peel Extract, Lavandula Angustifolia (Lavender) Leaf Extract, Sophora Angustifolia Root Extract, Oryza Sativa (Rice) Bran Extract, Rosa Canina Fruit Extract, Hamamelis Virginiana (Witch Hazel) Leaf Extract, Eucalyptus Globulus Leaf Extract, Crataegus Cuneata Fruit Extract, and Panax Ginseng Root Extract, and such.

These substances maintain or promote the self-renewal ability of epidermal stem cells. *In vivo* or in three-dimensional cultured epidermis, epidermal stem cells expressing sufficient amounts of COL17A1 competitively occupy and amplify the epidermis, competing with surrounding epidermal stem cells that express low amounts of COL17A1. Therefore, the self-renewal ability based on the expression of COL17A1 is called "competitive self-renewal ability". The self-renewal ability of epidermal stem cells, for example, can be referred to as the competitive self-renewal ability of epidermal stem cells, and the above substances can also be referred to as an "agent for competitive amplification of epidermal stem cells".

### Substances that inhibit genomic stress or oxidative stress in a cell

The present inventors also found that the expression of COL17A1 is decreased by various stresses.

In the present invention, the stresses to be suppressed are genomic stresses (genotoxic stresses) and oxidative stresses. Genomic stresses include genotoxic stresses such as DNA damage stress caused by ultraviolet rays, radiation stress, or anticancer drugs, or replication stress associated with cell division. Cell functions are all impaired by stresses that confer DNA damage or by blockage of self-renewal signals by molecularly targeted drugs. Therefore, it is understood by those skilled in the art that substances that inhibit genomic stresses or oxidative stresses in a cell or DNA damage in a cell can be used to promote and improve skin wound healing.

### Substances that inhibit DNA damage in a cell

For the substances that inhibit DNA damage, for example, Sarcandra glabra (Sarcandra glabra) extract, Saraca dives (Saraca dives) extract, Cudrania pubescens (Cudrania pubescens) extract, Taxodium distichum (Taxodium distichum) extract, Ludwigia octovalis (Ludwigia octovalis) extract, Deutzianthus tonkinensis (Deutzianthus tonkinensis) extract, Alchornea trewioides extract (Alchornea trewioides) extract, Berchemia polyphylla (Berchemia polyphylla) extract, Glochidion puberum (Glochidion puberum) extract, Sassafras tzumu (Sassafras tzumu) extract (see JP 2008-247854), Cinchona succirubra extract, Symphytum officinale extract, coffee tree extract, cassia extract, burdock extract, narcissus extract, Sophora Angustifolia extract, chlorella extract, Lavandula Angustifolia (Lavender) extract, stinging nettle extract, rose extract, amatyazuru extract, daylily extract, oleander extract, Panax Ginseng extract, linden extract, jute extract, lotus extract, tea extract, or okra extract (see WO2013/031003) can be used.

The DNA damage-inhibiting ability of a substance that inhibits DNA damage can be evaluated using any method known to those skilled in the art, such as a comet assay or an assay that detects the DNA damage focus induced by the DNA damage response. For the DNA damage-inhibiting substance, a substance that promotes repair of DNA damage may be used. For example, Gigartina tenella (Gigartina tenella) extract (see JP2006-273761) or cinchona extract (see WO2013/031003) can be used as an agent that promotes repair of DNA damage. The DNA damage repair ability of an agent that promotes DNA damage repair can be assessed using any method known to those skilled in the art, such as host-cell reactivation assay. Substances that inhibit a genomic stress or an oxidative stress in a cell include, but are not limited to, antioxidants, UV absorbers, UV scatterers, radioprotective agents, and promoters of DNA damage repair.

### Compositions for use in the inhibition of skin aging

One of the embodiments of the present invention relates to a composition for use in inhibiting skin aging, comprising as an active ingredient a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and/or a substance that inhibits DNA damage in a cell.

One of the embodiments of the present invention may be a composition for use in inhibiting epidermal aging, in particular. A part of the embodiment of the present invention may be a composition for use in the inhibition of aging of the skin that contains hair follicles, in particular. A part of the embodiment of the present invention may be a composition for use in the inhibition of aging of the skin that specifically excludes hair follicles.

The compositions for use in the suppression of skin aging comprises an active ingredient described above in the section "Compositions for use in the promotion of skin wound healing or in the prevention or improvement of skin ulcers or bedsores", and the active ingredients of the compositions may be applied in the same manner as described above below.

The skin's role in separating the individual from the outside world and its ability to regenerate are declined with age. The function of the skin is maintained by epidermal stem cells in the basal layer of the epidermis and their normal differentiation. The progeny cells of the epidermal stem cells adhere to the surface of the skin perpendicularly and align to form a layer with a certain thickness for normal keratinization, which keeps the skin's regenerative ability and maintains the strength as a barrier to the outside world to avoid formation of sores and ulcers. However, as the skin ages or undergoes various stresses, it begins to show common characteristics as changes that make it unable to fulfill these roles, and these changes are collectively referred to as skin aging.

With aging, the skin loses its fine texture and becomes uneven in appearance, resulting in fine lines and wrinkles. In addition, the epidermis, dermis, and/or lipid tissue become thinner or weaker, resulting in redness, eczema, sores, erosions, and ulcers. In addition, the intensity of pigments of the skin becomes more pronounced. In general, formation of wrinkles due to a decrease in collagen in the dermis, thinning (atrophy) of the epidermis, dermis, and lipid tissue, and loss of firmness and weakness are considered as characteristics of aging skin, and in addition, senile pigmentation, and depigmentation are well known. The present inventors have found that the common features of aging skin in mice and humans are histological atrophy of the epidermis, reduction of pigment cells, and reduction or immaturity of the hemidesmosome component of the basement membrane, especially decrease of COL17A1 and reduction of hemidesmosomes. Further, losses of fibroblasts in the upper dermis, and crepe wrinkles and fine lines due to skin dryness, as well as a decrease in the number of PDGFRα⁺ mesenchymal cells in the upper dermis were observed.

While it is known that the skin becomes weak when COL17A1 is deficient, the above findings may explain why in geriatric skin, wrinkles and erosions are more likely to occur, and ulcers are more likely to occur. Therefore, it is understood by those skilled in the art that substances that induce or maintain the expression of COL17A1 in a cell, inhibit the degradation of COL17A1 in a cell, inhibit genomic stress or oxidative stress in a cell, and/or inhibit DNA damage in a cell can be used to suppress skin aging.

One of the embodiments of the present invention is a composition for use against skin aging below:
(a) thinning, weakening, atrophy, or loss of firmness of the epidermis, dermis, and/or adipose tissue;
(b) decrease of the epidermal barrier function or dryness of the skin;
(c) reduction or immaturation of hemidesmosomes in the basement membrane;
(d) loss of fibroblasts in the upper dermis, or crepe wrinkles or fine wrinkles due to dryness of the skin;
(e) wrinkles due to loss of collagen in the dermis; and
(f) spots, senile pigment freckles, or depigmented spots.

One of the embodiments of the present invention is a composition for use in wrinkle inhibition, in particular.

One of the embodiments of the present invention relates to a composition for maintaining the regenerative ability or competitive self-renewal ability of epidermal stem cells, comprising as an active ingredient a substance that induces or maintains the expression of COL17A1 in a cell, a substance that inhibits the degradation of COL17A1 in a cell, a substance that inhibits genomic stress or oxidative stress in a cell, and/or a substance that inhibits DNA damage in a cell. In the context of the present specification, skin aging is accompanied by one or more of the following: thinning of the epidermis, reduction of the barrier function, dryness, weakening of the basement membrane area, wrinkle formation due to loss of collagen in the dermis, thinning, atrophy, loss of firmness, and weakness of the epidermis, dermis and/or lipid tissue, and spots or senile pigmentation. Epidermal stem cells are stem cells that reside in the basal layer of the epidermis and provide new epidermal keratinocytes, play an important role in skin metabolism and maintain the skin surface in a healthy state. When epidermal stem cells become aging, various genomic stresses, oxidative stresses, or rapid blockage of self-renewal signals by molecular targets, the intrinsic self-renewal ability of epidermal stem cells is reduced and they are easily lost through final differentiation.

As described above, the present inventors have shown that epidermal cell competition dynamics linked to COL17A1-mediated epidermal stem cell proliferation maintains skin homeostasis and thereby regulates skin organ aging. Epidermal stem cells with high COL17A1 expression have a high regenerative ability. Therefore, the skilled person understands that substances that induce or maintain the expression of COL17A1 in a cell, inhibit the degradation of COL17A1 in a cell, inhibit genomic or oxidative stress in a cell, and/or inhibit DNA damage in a cell can be used to enhance the regenerative ability of epidermal stem cells. By increasing the regenerative ability of epidermal stem cells and maintaining or improving the supply of new epidermal keratinocytes, skin aging is reduced and the surface condition of the skin is maintained or improved.

### Anti-wrinkle compositions

One of the embodiments of the present invention is a composition for use in anti-wrinkle treatment, comprising as an active ingredient a substance selected from the group consisting of a substance that induces or maintains the expression of COL17A1 in a cell, a substance that inhibits the degradation of COL17A1 in a cell, a substance that inhibits genomic stress or oxidative stress in a cell, and a substance that inhibits DNA damage in a cell. The anti-wrinkle effect is at least one selected from the group consisting of improvement of the skin elasticity, improvement of the stratum corneum function, improvement of the skin moisturizing ability, and improvement of the skin barrier function.

### Improvement of the skin elasticity

In the present invention, skin elasticity refers to mechanical properties such as flexibility (softness) and elasticity (the degree to which stretched skin returns to its original state like a spring within a certain period of time). Skin elasticity is considered to be one of the indicators of skin age; and moist, elastic, springy skin is perceived as "soft" and "firm". It is believed that ultraviolet rays affect the decline in skin elasticity, and the decline in elasticity is more pronounced in areas of exposure that are exposed to ultraviolet rays, including the face. Skin elasticity is also known as viscoelasticity, and can be determined quantitatively using Cutometer MPA580 (Courage + Khazaka, Integral Corporation, Germany) by measuring the process by which the skin returns to its original position after amplification of force to deform the skin. Cutometer is a commonly used instrument in the fields of dermatology and cosmetic science. Its principle is to measure viscoelasticity by applying negative pressure to the skin using a probe with a hole of 2 mm in diameter, suctioning the skin, and then releasing the negative pressure to measure the return of the skin. The values below are used for analysis, and it is thought that generally, the larger the value is, the better the elasticity is, and it decreases with age.

Ur/Uf value (R7): An index of elasticity expressed using the maximum elongation value (Uf or R0) and the height of elastic elongation at relaxation (Ur), which indicates the instantaneous rate of return of the skin to the maximum elongation.

Ur/Ue (R5): An index of elasticity expressed using the height of elastic elongation at relaxation (Ur) and the height of elastic elongation when sucked into the probe (Ue), which indicates the instantaneous rate of return of the skin to maximum elongation and is considered the net elasticity.

Ua/Uf (R2): An index of elasticity expressed using the maximum elongation value (Uf) and Ua, which indicates the instantaneous rate of return of the skin to the maximum elongation, and is also referred to as total elasticity. Ua is the difference between Uf and R1 (the height of residual strain after the first measurement cycle).

### Improvement of the stratum corneum function

Improvement of the stratum corneum function refers to, upon application to the skin, improvement of the moisturizing ability to increase or retain the stratum corneum water content, and improvement of the barrier function to inhibit the entry of foreign substances from the outside world; and an agent that improves the stratum corneum function is one that can exert either of these effects.

### Improvement of the skin moisturizing ability

In the present invention, "moisturizing ability" refers to the retention of stratum corneum water content; and the higher the stratum corneum water content is, the better the moisturizing ability is. An agent that improves the skin moisturizing ability is one that can retain the stratum corneum water content.

### Improvement of the skin barrier function

The "barrier function" refers to the suppression of transdermal moisture transpiration; and the smaller the transdermal moisture transpiration rate (TEWL) is, the better the barrier function is.

The skin barrier function prevents the evaporation of water from the body and the entry of foreign substances from outside the body, and this function is impaired by UV irradiation. In general, the stratum corneum water content, TEWL, and epidermal thickness are considered to be indicators of the skin barrier function, and are used to indicate the degree of a skin disorder caused by UV irradiation. An agent that improves the skin barrier function is one that is capable of either preventing water evaporation or preventing entry of foreign substances from outside the body.

### Improvement of skin roughness

Rough skin is generally defined as the loss of smoothness from the surface of the skin and the appearance of flakiness and problems, as opposed to healthy skin which is smooth and moisturized. It can cause superficial problems such as roughness, redness, unevenness, and even itching. It is known that rough skin is closely related to the decline of the skin barrier function. For this reason, a method to analyze and evaluate symptoms of skin roughness quantitatively by measuring TEWL, which is an indicator of the skin barrier function, has been used. Furthermore, since roughness (unevenness) of the stratum corneum surface occurs with skin roughness, skin roughness can be quantitatively analyzed from the skin surface roughness parameters of the ISO standard, such as arithmetic mean roughness (Ra), maximum height (Ry), and ten-point average roughness (Rz). The skin surface can be photographed using the three-dimensional high-resolution imaging system PRIMOS-CR, and the surface analysis can be performed using exclusive software (Primos OMC3_22). Using a skin roughness model that serves as a standard for chemical-induced skin damage in humans, the present inventors found that a substance that maintains the self-renewal ability of epidermal stem cells improves skin roughness, by suppressing the increase of TEWL associated with artificially induced skin roughness and improving the roughness of the skin surface.

Accordingly, one of the embodiments of the present invention provides a composition for use in improving rough skin, comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

Skin roughness is a condition in which the skin loses its smoothness, humidity, uneven appearance and texture, often accompanied by dryness, itching, redness, and erosion; and the compositions of the present invention can improve the above symptoms or conditions.

### Anti-spot compositions

The term "spot" refers to the deposits of melanin, a pigment produced in the skin. The most common type is "solar lentigo" (senile pigment freckle) caused by continuous exposure to ultraviolet rays, but it also refers to a wide range of other conditions in which the uniformity of pigmentation has been disrupted, such as ephelis (freckles), post-inflammatory hyperpigmentation, and melasma. Therefore, "anti-spot" means preserving the uniformity of pigmentation.

The present invention provides a composition for use in anti-spot treatment, comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

### Agent for prevention and improvement of a skin disorder caused by anticancer drugs

One of the embodiments of the present invention relates to methods and compositions for preventing or ameliorating skin disorders associated with anticancer drug therapy.

Skin damage (dermatotoxicity) occurs frequently as a side effect of treatment with anticancer drugs. In addition to skin dryness, hand-foot syndrome (palmar plantar erythrodysesthesia syndrome), pigmentation, nail changes, and aging-like skin disorders are known to occur. Conventional antimetabolites, alkylating agents, and platinum drugs have been used to cause skin dryness, atrophy, fragility, impaired barrier function, pigmentation abnormalities, and hair loss. In recent years, molecularly targeted drugs that selectively attack molecules specifically expressed in cancer cells have been developed based on the differences between cancer cells and normal cells, and they are being used in various cancers such as breast cancer and lung cancer, replacing conventional anticancer drugs.

In addition to skin disorders commonly observed with anticancer agents (dry skin, atrophy, hand-foot syndrome, pigmentation abnormalities, alopecia, etc.), erythematous papules, acne-like skin eruptions, seborrheic dermatitis, hair and nail hyperpigmentation, onychomycosis, skin dryness, fissuring, xerosis, pruritus, and intense burning sensation due to skin toxicity caused by molecularly targeted agents (various multi-tyrosine kinase inhibitors, EGF receptor inhibitors, etc.) are frequently observed. New immunotherapeutic agents (immune checkpoint inhibitors) have also been found to cause skin disorders. Skin lesions caused by these molecularly targeted drugs are not easily regarded as harmful, but rather as an indication of pharmacological effects on skin a cell that express molecules common to cancer cells, or as an indicator of the efficacy of anticancer drugs; and anticancer therapy is continued while maintaining a symptomatic treatment such as moisturizers. However, it causes a great deal of psychological distress and burden to patients, and there are many cases where patients give up the treatment themselves. In addition, there are many cases where severe skin damage leads to the discontinuation of anticancer drugs (chemotherapy and molecularly targeted drugs), but there is no appropriate preventive method or local treatment method.

To date, it has been unclear whether the expression of COL17A1 in epidermal stem cells is altered by various anticancer drugs, whether its expression is reduced by molecularly targeted drugs or how it is suppressed, or whether it is related to anticancer drugs. Although symptomatic treatments such as applying moisturizers and steroids to the skin have been performed in trial and error, no effective method has been developed to prevent or ameliorate skin disorders associated with cancer treatment.

In the present invention, it was found that not only anticancer drugs such as hydroxyurea but also molecularly targeted drugs such as inhibitors of the EGF receptor reduce the expression of COL17A1 and the competitive self-renewal ability of stem cells.

Therefore, the present invention provides a composition for use in preventing or improving a skin disorder caused by an anticancer agent, comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

In one of the embodiments of the present invention, the agent for preventing or ameliorating skin disorders may be characterized in that it contains as an active ingredient a substance that maintains the expression of COL17A1 and the self-renewal ability in a cell. More specifically, the agent for preventing or improving skin disorders may include a ROCK inhibitor, an NADPH oxidase inhibitor, an iNOS inhibitor, and/or a COX inhibitor as a substance that induces or maintains the expression of COL17A1 in a cell. More specifically, the agent for prevention and amelioration of skin disorders of the present invention may include apocynin, ebselen, celecoxib, apigenin, Y-27632, Ripasudil, 1400W, astaxanthin, ethinylestradiol, biochanin A, Necrostatin 1, Apocynum Venetum Leaf/Stem Extract, Morus Alba Leaf Extract, Gymnema Sylvestre Leaf Extract, Camellia Sinensis Leaf Extract, Eriobotrya Japonica Leaf Extract, Silybum Marianum Extract, Kaempferia Parviflora Extract, Plantago Major Seed Extract, Coffea Arabica (Coffee) Seed Extract, Prunus Yedoensis Leaf Extract, Melia Azadirachta Leaf Extract, Citrus Nobilis (Tangerine) Peel Extract, Lavandula Angustifolia (Lavender) Leaf Extract, Sophora Angustifolia Root Extract, Oryza Sativa (Rice) Bran Extract, Rosa Canina Fruit Extract, Hamamelis Virginiana (Witch Hazel) Leaf Extract, Eucalyptus Globulus Leaf Extract, Crataegus Cuneata Fruit Extract, and Panax Ginseng Root Extract, and such.

### Plant extracts

In the present invention, various plant extracts can be used for the applications described above (below).
- Promotion of skin wound healing, or prevention or improvement of skin ulcer
- Inhibition of skin aging
- Enhancement of the regenerative ability of epidermal stem cells
- Prevention or improvement of a skin disorder caused by an anticancer drug
- Anti-wrinkle treatment (improvement of the skin elasticity, improvement of the stratum corneum function, improvement of the skin moisturizing ability, and improvement of the skin barrier function)
- Anti-spot treatment

Furthermore, in the present invention, the plant extracts can also be used as COL17A1 promoters and inhibitors of hair loss and graying.

The raw materials for plant extracts used in the present invention include Apocynum venetum (Apocynum venetum), mulberry (Morus alba), gymnema (Gymnema sylvestre), tea (Camellia sinensis), maria thistle (Silybum marianum), loquat leaf (Eriobotrya japonica), black turmeric (Kaempferia parviflora), oilseed rape (Panax ginseng), and ashwagandha (Indian ginseng). The parts of these plants used are not limited as long as the above effects can be obtained.

The extraction method of each plant extract is not particularly limited and can be performed according to the methods known to those skilled in the art. As the extraction solvent, water, alcoholic solvents, and organic solvents such as acetone, esters, polyhydric alcohols, and ethers can be used. Thus, for example, ethanol extracts, hot water extracts, and 1,3-butylene glycol extracts of the above plant materials can be prepared. These solvents may be used alone or in combination. The amount of the extraction solvent, extraction temperature, extraction time, and extraction method are not limited as long as it is possible to obtain the composition that exhibits the above effects.

The extract may be the filtrate itself obtained by filtering the obtained extract, a concentrated liquid obtained by concentrating the filtrate, a dried product obtained by drying the concentrated liquid, and a crude or purified product thereof. Any concentration method or any drying method can be used. If necessary, excipients such as dextrin may be added. Purification can be carried out according to the means known to those skilled in the art, such as synthetic adsorption resins, activated carbon, ion exchange resins, column chromatography, and recrystallization.

In addition, the composition can take the form of a food composition, a pharmaceutical composition, or a cosmetic composition; and in particular, in the case of a food composition, it can take the form of a functional food or health food.

### Pharmaceutical compositions

The compositions of the present invention may be pharmaceutical compositions. One of the embodiments of the present invention relates to a pharmaceutical composition for use in promoting skin wound healing or preventing or improving skin ulcers or bedsores, comprising as an active ingredient a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and/or a substance that inhibits DNA damage in a cell.

The pharmaceutical compositions of the present invention may be used in the treatment of acute skin wounds or in the prevention or treatment of chronic skin wounds. In other words, one of the embodiments of the present invention relates to the use of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and/or a substance that inhibits DNA damage in a cell in the production of a medicament for use in promoting skin wound healing or preventing or improving skin ulcers or bedsores.

One of the embodiments of the present invention relates to a pharmaceutical composition comprising as an active ingredient a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and/or a substance that inhibits DNA damage in a cell, wherein the pharmaceutical composition is for use in the inhibition of skin aging.

The pharmaceutical compositions of the present invention can be used for the prevention or treatment of skin manifestations of diseases associated with the progression of skin aging. In other words, one of the embodiments of the present invention relates to the use of a substance that induces or maintains the expression of COL17A1 in a cell, inhibits the degradation of COL17A1 in a cell, maintains the self-renewal ability of epidermal stem cells, inhibits genomic stress or oxidative stress in a cell, and/or a substance that inhibits DNA damage in a cell in the manufacture of a medicament for use in the inhibition of skin aging.

One of the embodiments of the present invention relates to a pharmaceutical composition comprising as an active ingredient, a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and/or a substance that inhibits DNA damage in a cell, wherein the pharmaceutical composition is for use in enhancing the regenerative ability of epidermal stem cells.

The pharmaceutical compositions of the present invention can be used for the prevention or treatment of skin manifestations of diseases associated with reduced regenerative ability of epidermal stem cells. In other words, one of the embodiments of the present invention relates to the use of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and/or a substance that inhibits DNA damage in a cell in the manufacture of a medicament for enhancing the regenerative ability (self-renewal ability) of epidermal stem cells, or in the manufacture of regenerative medical products (processed cells, processed cell sheets, etc.).

Furthermore, one of the embodiments of the present invention relates to a pharmaceutical composition comprising as an active ingredient, a substance that resists a molecularly targeted drug that impairs the regenerative ability (self-renewal and maintenance) of epidermal stem cells, a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and/or a substance that inhibits DNA damage in a cell.

One of the embodiments of the present invention also relates to a pharmaceutical composition for use in preventing or ameliorating a skin disorder caused by an anticancer drug, comprising as an active ingredient a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and/or a substance that inhibits DNA damage in a cell.

The pharmaceutical compositions of the present invention may be used in the prevention or amelioration of a skin disorder caused by an anticancer agent. In other words, one of the embodiments of the present invention relates to the use of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and/or a substance that inhibits DNA damage in a cell in the manufacture of a medicament for use in the prevention or amelioration of a skin disorder caused by an anticancer agent.

One of the embodiments of the present invention is a pharmaceutical composition for use in anti-wrinkle, characterized in that it contains as an active ingredient a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and/or a substance that inhibits DNA damage in a cell. A pharmaceutical composition characterized in that it contains, as an active ingredient, a substance that inhibits genomic stress or oxidative stress in a cell and/or a substance that inhibits DNA damage in a cell

The pharmaceutical compositions of the present invention can be used for anti-wrinkle treatment. In other words, one of the embodiments of the present invention relates to the use of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and/or a substance that inhibits DNA damage in a cell in the manufacture of a medicament for use in anti-wrinkle treatment.

One of the embodiments of the present invention relates to a pharmaceutical composition comprising as an active ingredient, a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and/or a substance that inhibits DNA damage in a cell, wherein the pharmaceutical composition is for use in the improvement of rough skin.

The pharmaceutical compositions of the present invention can be used to improve skin roughness. In other words, one of the embodiments of the invention relates to the use of a substance that induces or maintains the expression of COL17A1 in a cell, a substance that inhibits the degradation of COL17A1 in a cell, a substance that maintains the self-renewal ability of epidermal stem cells, a substance that inhibits genomic or oxidative stress in a cell, and/or a substance that inhibits DNA damage in a cell in the manufacture of a medicament for use in the improvement of rough skin.

The pharmaceutical compositions of the present invention can be used for anti-spot treatment. In other words, one of the embodiments of the present invention relates to the use of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and/or a substance that inhibits DNA damage in a cell in the manufacture of a medicament for use in anti-spot treatment.

The substances that act as active ingredients in the pharmaceutical compositions of the present invention include, for example, ROCK inhibitors, NADPH oxidase inhibitors, iNOS inhibitors, COX inhibitors, and ELANE inhibitors as described in other parts of the specification, and more specifically, Y27632, apocynin, and sivelestat, and such, but they are not limited thereto.

The pharmaceutical composition of the present invention may take any form, such as tablet, powder, liquid, and semi-solid. The pharmaceutical composition of the present invention may also be a topical agent, such as an ointment applied through the skin or used for local treatment added directly to the skin lesion; and it may be prepared by blending various principal agents into a base formulation. In addition to the above active ingredients, the pharmaceutical composition of the present invention may comprise a pharmaceutically acceptable excipient, an additive, a buffer, a salt for isotonic adjustment, an antioxidant, a preservative, or a pharmaceutical stabilizer. Examples of an excipient include, but are not limited to, water, purified water, alcohol, glycerin, lactose, starch, dextrin, white sugar, precipitated silica, honey, rice starch, and tragacanth. The pharmaceutical composition of the present invention may also contain other active ingredients. The amount of each component may be determined as appropriate within the range acceptable for pharmaceutical use. The dosage of the composition can be determined as appropriate according to the type of drug to be used and the subject to be administered. For example, the active ingredient may be 0.01-15% by weight, for example, 0.1-5% by weight.

The route of administration can also be determined according to the type of drug used and the subject to be administered. The pharmaceutical composition of the present invention may be included in a wound dressing material such as a dressing material. A dressing material is a medical material capable of maintaining a moist environment and providing an optimal environment for wound healing.

One of the embodiments of the present invention relates to a method for promoting skin wound healing in a mammal, a method for preventing or ameliorating a skin ulcer or bedsore, a method for inhibiting skin aging, a method for enhancing the regenerative ability of epidermal stem cells, a method for preventing or ameliorating a skin disorder caused by an anticancer agent, an anti-wrinkle method, an anti-spot method, and/or a method for improving rough skin.

Such methods may include the steps of inducing or maintaining the expression of COL17A1 in a cell, inhibiting the degradation of COL17A1 in a cell, inhibiting genomic or oxidative stress in a cell, and/or inhibiting DNA damage in a cell. More specifically, one of the embodiments of the present invention may include administering to a mammal, for example, a ROCK inhibitor, an NADPH oxidase inhibitor, an iNOS inhibitor, and/or a COX inhibitor.

Mammals include, for example, humans, monkeys, mice, rats, rabbits, dogs, cats, sheep, goats, alpacas, horses, cattle, pigs, mink, foxes, marten, raccoons, chinchillas, otters, otters, beavers, and seals. The dosage can be determined according to the type of drug to be used and the subject to be administered (same as above). The route of administration can also be determined according to the type of drug to be used and the subject to be administered. Preferred routes of administration include, for example, application or spraying of a liquid, lotion, or cream formulation on the skin, or subcutaneous injection of a liquid formulation, or oral administration of a solid formulation or liquid formulation. A patch containing the composition of the present disclosure may be prepared and applied to the skin.

Agents for the prevention and improvement of pigmentation abnormalities

One of the embodiments of the present invention relates to methods and compositions for preventing or ameliorating pigmentation abnormalities in aging skin. Such methods may include the steps of inducing or maintaining the expression of COL17A1 in a cell, inhibiting the degradation of COL17A1 in a cell, inhibiting genomic or oxidative stress in a cell, and/or inhibiting DNA damage in a cell. Such compositions may also include a substance that induces or maintains the expression of COL17A1 in a cell, a substance that inhibits the degradation of COL17A1 in a cell, a substance that inhibits genomic or oxidative stress in a cell, and/or a substance that inhibits DNA damage in a cell.

One of the embodiments of the present invention relates to methods and compositions for preventing or improving crepe wrinkles, fine lines, dryness and sores due to age-related changes in the skin. Such methods may include the steps of inducing or maintaining the expression of COL17A1 in a cell, inhibiting the degradation of COL17A1 in a cell, inhibiting genomic or oxidative stress in a cell, and/or inhibiting DNA damage in a cell. Such compositions may also include a substance that induces or maintains the expression of COL17A1 in a cell, a substance that inhibits the degradation of COL17A1 in a cell, a substance that inhibits genomic or oxidative stress in a cell, and/or a substance that inhibits DNA damage in a cell.

### Regulators of stem cell competition

One of the embodiments of the present invention relates to methods and compositions for use in regulating cell competition in epidermal stem cells. The present inventors have found that COL17A1 is an important factor involved in cell competition. Accordingly, the methods of the present invention may include the steps of inducing or maintaining the expression of COL17A1 in a cell, inhibiting the degradation of COL17A1 in a cell, inhibiting genomic or oxidative stress in a cell, and/or inhibiting DNA damage in a cell. The compositions may also include a substance that induces or maintains the expression of COL17A1 in a cell, a substance that inhibits the degradation of COL17A1 in a cell, a substance that inhibits genomic or oxidative stress in a cell, and/or a substance that inhibits DNA damage in a cell. It is also understood by those skilled in the art that cell competition can also be negatively regulated by inhibiting COL17A1.

### Beauty supplements or functional foods

One of the embodiments of the present invention relates to a beauty supplement or functional food containing a substance that can maintain the expression of COL17A1 in a cell and the competitive self-renewal ability based on the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and/or a substance that inhibits DNA damage in a cell. In one of the embodiments, the beauty supplement or functional food product of the present invention may be characterized in that it comprises as an active ingredient, a substance that induces or maintains the expression of COL17A1 in a cell. More specifically, the beauty supplement or functional food product of the present invention may include a ROCK inhibitor, an NADPH oxidase inhibitor, an iNOS inhibitor, and/or a COX inhibitor as a substance that induces or maintains the expression of COL17A1 in a cell. Even more specifically, the beauty supplements or functional foods of the present invention may comprise, as a substance that maintains the expression of COL17A1 in a cell and maintains the self-renewal ability, for example, apocynin, ebselen, celecoxib, apigenin, Y-27632, Ripasudil, 1400W, astaxanthin, ethinylestradiol, biochanin A, Necrostatin 1, Apocynum Venetum Leaf/Stem Extract, Morus Alba Leaf Extract, Gymnema Sylvestre Leaf Extract, Camellia Sinensis Leaf Extract, Eriobotrya Japonica Leaf Extract, Silybum Marianum Extract, Kaempferia Parviflora Extract, Plantago Major Seed Extract, Coffea Arabica (Coffee) Seed Extract, Prunus Yedoensis Leaf Extract, Melia Azadirachta Leaf Extract, Citrus Nobilis (Tangerine) Peel Extract, Lavandula Angustifolia (Lavender) Leaf Extract, Sophora Angustifolia Root Extract, Oryza Sativa (Rice) Bran Extract, Rosa Canina Fruit Extract, Hamamelis Virginiana (Witch Hazel) Leaf Extract, Eucalyptus Globulus Leaf Extract, Crataegus Cuneata Fruit Extract, and Panax Ginseng Root Extract, or such.

The beauty supplements or functional foods of the present invention may take any form, such as tablet, powder, semi-solid, jelly or liquid. The beauty supplements of the present invention may further comprise at least one of a skin anti-aging agent, vitamin, collagen and mineral. The beauty supplements or functional foods of the present invention may be prepared, for example, to be taken orally one to three times a day, before, during, or after meals.

### Cosmetic compositions

One of the embodiments of the present invention relates to a cosmetic composition, comprising as an active ingredient a substance that can maintain the expression of COL17A1 in a cell and the competitive self-renewal ability based thereon, a substance that inhibits the degradation of COL17A1 in a cell, a substance that maintains the self-renewal ability of epidermal stem cells, a substance that inhibits genomic stress or oxidative stress in a cell, and/or a substance that inhibits DNA damage in a cell. The substances that induce or maintain the expression of COL17A1 in a cell are as described in other parts of the present specification and are, for example, ROCK inhibitors, NADPH oxidase inhibitors, iNOS inhibitors, and COX inhibitors, without being limited thereto. For the substance that induces or maintains the expression of COL17A1 in a cell, for example, apocynin, ebselen, celecoxib, apigenin, Y-27632, Ripasudil, 1400W, astaxanthin, ethinylestradiol, biochanin A, Necrostatin 1, Apocynum Venetum Leaf/Stem Extract, Morus Alba Leaf Extract, Gymnema Sylvestre Leaf Extract, Camellia Sinensis Leaf Extract, Eriobotrya Japonica Leaf Extract, Silybum Marianum Extract, Kaempferia Parviflora Extract, Plantago Major Seed Extract, Coffea Arabica (Coffee) Seed Extract, Prunus Yedoensis Leaf Extract, Melia Azadirachta Leaf Extract, Citrus Nobilis (Tangerine) Peel Extract, Lavandula Angustifolia (Lavender) Leaf Extract, Sophora Angustifolia Root Extract, Oryza Sativa (Rice) Bran Extract, Rosa Canina Fruit Extract, Hamamelis Virginiana (Witch Hazel) Leaf Extract, Eucalyptus Globulus Leaf Extract, Crataegus Cuneata Fruit Extract, and Panax Ginseng Root Extract, or such can be used.

In one of the embodiments, a cosmetic composition according to the present disclosure is a composition for use in skin care. In one of the embodiments, the cosmetic composition of the present disclosure relates to a composition for application to the surface of skin. The compositions can be in a variety of product forms including solutions, suspensions, lotions, creams, gels, lotions, sticks, pencils, sprays, aerosols, ointments, cleansing detergent solutions and cleansing stick solids, shampoos and hair conditioners, pastes, foams, powders, mousses, shaving creams, wipes, strips, patches, electric patches, wound dressings and adhesive bandages, hydrogels, film-forming products, face and skin masks (with and without insoluble sheets), and makeup products such as foundations, eyeliners, and eye shadows, without being limited thereto.

The cosmetic compositions of the present disclosure may contain 0.01 -15% by weight, for example, 0.1 - 5% by weight of a substance that induces or maintains the expression of COL17A1 in a cell (Y27632, apocynin, or such).

The cosmetic compositions of the present disclosure may further comprise at least one of an anti-skin aging agent, a skin tone-adjusting agent, an anti-inflammatory agent, and a suncreen agent. Examples of an anti-skin aging agent include, but are not limited to, peptides having anti-aging properties (WO2014157485A1). Suitable skin tone-adjusting agents include, but are not limited to, sugar amines, arbutin, deoxyarbutin, hexylresorcinol, kojic acid, hexamidine compounds, salicylic acid, and retinoids, including retinol propionate and retinyl propionate.

Suitable anti-inflammatory agents include non-steroidal anti-inflammatory drugs (NSAIDS such as ibuprofen, naproxen, and such), glycyrrhizic acid (also known as glycyrrhetinic acid glycoside), and salts such as dipotassium glycyrrhizate, glycyrrhetenic acid (glycyrrhetenic acid), licorice extract, bisabolol (e.g., alpha bisabolol), manjista (extracted from plants of the genus Acanthopanax, specifically (Rubia cordifolia)), and guggal (guggal) (extracted from plants of the genus Commiphora, especially Commiphora mukul)), koranoki extract, chamomile, purple clover extracts, and sea whip extracts (extracts from the plants of the Gorgonacea Order), derivatives of any of the above, and mixtures thereof, but are not limited thereto.

Suitable sunscreen agents include 2-ethylhexyl-p-methoxycinnamate, 4,4'-t-butylmethoxydibenzoylmethane, 2-hydroxy-4-methoxybenzophenone, octyldimethyl-p-aminobenzoic acid, digalloyl trioleate, 2,2 dihydroxy-4-methoxybenzophenone, ethyl-4-(bis(hydroxypropyl)aminobenzoate, 2-ethylhexyl-2-cyano-3,3-diphenylacrylate, 2-ethylhexyl salicylate, glyceryl-p-aminobenzoate, 3,3,5-tri-methylcyclohexyl salicylate, methyl anthranilate, p-dimethyl-aminobenzoate or aminobenzoate, 2-ethylhexyl-p-dimethylaminobenzoate, 2-phenylbenzimidazole-5-sulfonic acid, 2-(p-dimethylaminophenyl)-5-sulfonic benzoxazoic acid, octocrylene, zinc oxide, benzylidene camphor and its derivatives, titanium dioxide, and mixtures thereof, but are not limited thereto.

The cosmetic compositions of the present disclosure may comprise at least one type of additive conventionally used in the cosmetic field that does not affect the properties of the compositions of the present disclosure, such as thickening agents, fragrances, pearlescent agents, preservatives, sunscreen agents, anionic or nonionic or cationic or amphoteric polymers, proteins, protein hydrolysates, for example, 18-methyl eicosanoic acid, vitamins, panthenol, silicones, fatty acids such as vegetable oil, animal oil, mineral oil or synthetic oil, gelling agents, antioxidants, solvents, filters, screening agents, odor absorbers, coloring agents, abrasives, absorbents, cosmetic ingredients such as aromatic agents, dyes, pigments/coloring agents, essential oils, anticaking agents, antifoaming agents, antimicrobial agents, binding agents, biological additives, buffers, fillers, chelating agents, chemical additives, cosmetic astringents, cosmetic biocides, denaturants, drug astringents, skin softeners, topical analgesics, film forming agents or materials, emulsifiers, pH adjusters, preservatives, atomizers, reducing agents, scavengers, skin cooling agents, skin protectants, thickening agents, viscosity modifiers, vitamins, and combinations thereof. These additives may be present in the compositions according to the present disclosure in proportions which, with respect to the total weight of the composition, preferably or advantageously fall within the range of 0 to 50% by weight, 5 to 40% by weight or 30 to 50% by weight.

The cosmetic compositions of the present disclosure, for topical application to the skin, may be, in particular, aqueous or oily solutions, dispersions of the lotion or serum type, emulsions with a liquid or semi-liquid consistency of the milk type obtained by dispersion of the fatty phase in the aqueous phase (O/W) or vice versa (W/O), and suspensions or emulsions with a soft consistency of the aqueous or anhydrous gel, and may additionally have microcapsules or microparticles, vesicular dispersions of the ionic and/or nonionic type, or foamy forms. These compositions are prepared according to conventional methods. It is preferable that the compositions have an aqueous phase of between 5 and 99.5%.

The pH of the cosmetic compositions of the present disclosure is generally, but not limited to, between 2 and 12, and preferably between 3 and 9. The pH can be prepared to the target value, for example, by adding ammonia, sodium hydroxide, potassium hydroxide, or a base (organic or inorganic) of primary, secondary, or tertiary (poly)amine, such as monoethanolamine, diethanolamine, triethanolamine, isopropanolamine, or 1,3-propanediamine, or, a basic amino acid or a polyamino acid such as lysine or arginine, alternatively, an inorganic or organic acid, and preferably a carboxylic acid such as citric acid, for example.

The cosmetic compositions of the present disclosure are, in particular, creams for cleansing, protecting, treating or caring for the face, hands, feet, folds on major body structures or the body (e.g., day cream, night cream, anti-aging cream, moisturizing cream, make-up removing cream, foundation creams, or sun creams), liquid foundations, make-up removing milks, protective or caring body milks, sun milks, lotions, gels or foams for caring of the skin, for example, cleansing lotions, sun lotions, artificial tanning lotions, bathing compositions, deodorant compositions containing disinfectants, aftershave gels or lotions, hair removal creams, and compositions for dealing with insect bites.

The cosmetic compositions of the present disclosure may be applied at least once a day, twice a day, or more frequently throughout the treatment period. When it is applied twice a day, there should be an interval of at least 1 to 12 hours between the first and second application, for example. Typically, the composition can be applied in the morning and/or at night before bedtime.

### Screening method

It relates to a method of screening for a substance that is effective in promoting skin wound healing or preventing or improving skin ulcers, inhibiting skin aging, regulating cell competition, improving (enhancing, augmenting) the regenerative ability of epidermal stem cells, preventing or improving a skin disorder caused by an anticancer agent, anti-wrinkle treatment, anti-spot treatment, and/or improving skin roughness. Herein, the inhibition of skin aging may be the inhibition of aging of hair follicles, which are appendages of the skin. Such a screening method may include the steps i - iii below:
(i) contacting the cells with a test substance *in vitro;*
(ii) measuring the expression of COL17A1 in the cells; and
(iii) determining whether the test substance upregulates the expression of COL17A1.

Here, the measurement of the expression of COL17A1 can be performed using any method known to those skilled in the art. The substances identified by the screening method described above may be used in the present invention as an ingredient for promoting skin wound healing, an ingredient for inhibiting skin aging, an ingredient for regulating cell competition, an ingredient for enhancing the regenerative ability of epidermal stem cells, an ingredient for preventing or improving a skin disorder caused by an anticancer agent, an anti-wrinkle ingredient, an anti-spot ingredient, and/or an ingredient for improving rough skin. Also, in the present invention, they are also useful as a substance to be administered to a mammal in the methods for promoting skin wound healing, the methods for inhibiting skin aging, the methods for enhancing the regenerative ability of epidermal stem cells, the methods for preventing or improving a skin disorder caused by an anticancer agent, the anti-wrinkle methods, the anti-spot methods, and/or the methods for improving skin roughness in the mammal.

The above screening methods may further comprise a step of determining whether the test substance promotes colony formation. By combining assessment of the COL17A1 expression with the colony formation assay, it is possible to screen for agents that promote "stem cell amplification via stem cell competition", by assessing agents that maintain the self-renewal ability through maintenance of the continuous expression of COL17A1. That is, in one of the embodiments, the screening methods may comprise the following steps i-iv:
(i) contacting cells with a test substance in vitro;
(ii) measuring the expression of COL17A1 in the cells;
(iii) determining whether the test substance upregulates the expression of COL17A1; and
(iv) determining whether the test substance promotes colony formation.

### Skin aging evaluation method and assessment kit

One of the embodiments of the present invention relates to a method of evaluating skin aging using the expression of COL17A1 in a cell as an indicator. The present inventors have found that the expression of COL17A1 is decreased in aged epidermal cells. Therefore, COL17A1 can be used as a marker for evaluating skin aging, and skin aging can be evaluated by measuring the expression of COL17A1 in epidermal cells. The expression of COL17A1 in epidermal cells can be measured by any method known to those skilled in the art, such as Western blotting or antibody staining using an antibody against COL17A1 (an anti-COL17A1 antibody), and mRNA measurement by RT-PCR of the gene encoding COL17A1. The results of the measurements can be compared with controls or over time to evaluate skin aging. For example, if the expression level of COL17A1 is decreased as a result of the comparison, skin aging is considered to be in progress.

One of the embodiments of the present invention also relates to a kit for use in a method for evaluating skin aging, using the expression of COL17A1 in a cell as an indicator. The kit comprises, for example, an antibody that specifically recognizes COL17A1. Such an antibody may be directly labeled, or it may be recognized by a labeled secondary antibody. The labeling can be, for example, based on the color development by an enzymatic activity, or chemiluminescence. The kit may also comprise, for example, oligonucleotides (probes, primers) for detecting the transcript of COL17A1. Such oligonucleotides can be used for detection by hybridization with the transcript or in amplification reactions such as PCR. Thus, one of the embodiments of the present invention relates to a kit for use in a method for evaluating skin aging, comprising an antibody for detecting COL17A1 (an anti-COL17A1 antibody) and a probe or primer for a gene encoding COL17A1. In addition, the kit may also include components (antibodies, probes, primers, etc.) for measuring the expression levels of other marker proteins such as p16Ink4a.

### Epidermal stem cell evaluation method and evaluation kit

One of the embodiments of the present invention relates to a method of evaluating epidermal stem cells using the expression of COL17A1 in a cell as an indicator. The present inventors have found that there is a correlation between the proliferative ability of epidermal stem cells and the expression level of COL17A1. Thus, it is possible to use COL17A1 as a marker for evaluating epidermal stem cells, and epidermal stem cells can be evaluated by measuring the expression of COL17A1 in epidermal stem cells. Epidermal stem cells expressing high levels of COL17A1 can be assessed to be cells suitable for use in culture because they have high proliferative potentials. The expression of COL17A1 in epidermal stem cells can be measured by any method known to persons skilled in the art, such as Western blotting, antibody staining, ELISA, FACS, and such using anti-COL17A1 antibodies. The present inventors have also found that the expression of COL17A1 is decreased in aged epidermal stem cells. Therefore, the aging of epidermal stem cells can be evaluated by measuring the expression level of COL17A1 in epidermal stem cells. Epidermal stem cell aging refers to the phenomenon in which the original self-renewal ability of epidermal stem cells is reduced by aging as well as various genomic stresses and oxidative stresses, and is easily lost through final differentiation. For example, if the expression level of COL17A1 is considered to be high in comparison with a standard value, the cells are considered to be suitable for use in culture and regenerative medicine products.

One of the embodiments of the present invention also relates to a kit for use in a method for evaluating epidermal stem cells, using the expression of COL17A1 in the cells as an indicator. The kit can include, for example, an antibody that specifically recognizes COL17A1, a probe for a gene encoding COL17A1, or primers for amplification of a gene encoding COL17A1. Thus, one of the embodiments of the present invention relates to a kit for use in a method for evaluation of epidermal stem cells, which comprises an antibody, probe or primer for detecting COL17A1.

### Epidermal stem cell sorting method and sorting kit

One of the embodiments of the present invention relates to a method of sorting epidermal stem cells using the expression of COL17A1 in the cells as an indicator. The present inventors have found that there is a correlation between the proliferative potential of epidermal stem cells and the expression level of COL17A1. Epidermal stem cells expressing high levels of COL17A1 can be assessed to be cells suitable for use in culture because they have high proliferative potentials. Therefore, COL17A1 can be used as a marker to evaluate the proliferative potential of epidermal stem cells, and measuring the COL17A1 expression in epidermal stem cells and selecting epidermal stem cells with high COL17A1 expression may be useful for subsequent culture and transplantation. The selection of cells with high COL17A1 expression can be performed by any method known to those skilled in the art, such as a method that uses FACS.

One of the embodiments of the present invention also relates to a kit for use in a method of sorting epidermal stem cells using the expression of COL17A1 in the cells as an indicator. The kit can include, for example, an antibody, probe or primer that specifically recognizes COL17A1, as described above. Thus, one of the embodiments of the present invention relates to a kit for use in a method of sorting epidermal stem cells, comprising an antibody, probe or primer for detecting COL17A1.

### Epidermal stem cell and/or epidermal cell amplification method and evaluation kit

One of the embodiments of the present invention also relates to a method of amplifying epidermal stem cells and/or epidermal cells, comprising the step of selecting epidermal stem cells having a high expression level of COL17A1. Since epidermal stem cells having a high expression level of COL17A1 have high proliferative potentials, epidermal stem cells and/or epidermal cells can be efficiently amplified by selecting such cells. Epidermal stem cells with high expression of COL17A1 mean that their expression level is quantitatively higher than that in aged cells. For example, they can be isolated by methods that use FACS or methods that use beads. Such amplification methods can be used to produce cell sheets *ex vivo.*

One of the embodiments of the present invention also relates to a kit for use in a method of amplifying epidermal stem cells and/or epidermal cells, which comprises a step of sorting epidermal stem cells having a high expression level of COL17A1. The kit can include, for example, an antibody, probe or primer that specifically recognizes COL17A1 as described above. Thus, one of the embodiments of the present invention relates to a kit for use in a method of amplifying epidermal stem cells and/or epidermal cells, which comprises an antibody, probe or primer for detecting COL17A1.

### Cell competition evaluation method and evaluation kit

One of the embodiments of the present invention relates to a method for evaluating molecules involved in cell competition, substances that promote or reduce cell competition, or substances that change the efficiency or speed of cell competition, by altering the expression levels of molecules that modify the expression of molecules involved in cell competition, such as COL17A1, in cells. The present inventors have found that the expression level of COL17A1 correlates with cell competition in epidermal stem cells. Epidermal stem cells with a high expression level of COL17A1 are advantageous in cell competition. The evaluation of cell competition can be performed using three-dimensional cultures of epithelial cells that form stratified squamous epithelium, such as keratinocytes (including cultured keratinocytes, the keratinocyte lines of HaCaT cells and Pam212 cells). The cells with a high expression level of COL17A1 proliferate better than the cells with a low expression level of COL17A1, and can be evaluated as having high cell competitive ability.

In the present invention, cell competition can be evaluated in three-dimensional culture of keratinocytes, and compounds, extracts, and nucleic acids that control this competition can be searched for. Thus, one of the embodiments of the present invention also relates to a method of evaluating cell competition in keratinocytes, and in particular to a method of screening for substances that control cell competition, using cell competition in three-dimensional culture of keratinocytes (line) as an indicator.

One of the embodiments of the present invention also relates to a kit for use in a method for evaluating cell competition in cornified cells (keratinocytes). The kit can include, for example, nucleic acids that alter the expression of cell competition-related molecules such as COL17A1 and cancer-related genes, for example, nucleic acids for use in RNA interference (siRNA, shRNA, and such against a gene encoding COL17A1), as well as gene transfer reagents, lentiviruses and retroviruses, antibodies for immunostaining such as antibodies that specifically recognize COL 17A1, antibodies used for immunostaining such as antibodies that specifically recognize COL17A1, or devices for three-dimensional culture. Thus, one of the embodiments of the present invention relates to a kit for use in a method for evaluating cell competition, which comprises cell lines, viruses, antibodies, and devices involved in the evaluation of cell competition in keratinocytes. The kit may also include a keratinocyte cell line (for example, HaCaT cells) for performing three-dimensional culture.

### Cell composition

One of the embodiments of the present invention relates to a cell composition (cell population) comprising isolated, COL17A1-expressing cells. Isolation of COL 17A1-expressing cells can be performed by any method known to those skilled in the art, such as methods that use FACS or beads. The cell compositions of the present invention may also comprise epithelial sheets, organoids, and such having a three-dimensional structure.

It is desirable that the cells in the cell composition of the present invention express high levels of COL17A1. Thus, cells expressing high levels of COL17A1 (COL17A1^{high} cells) can be isolated using, for example, FACS. Here, the terms "high" or "hi" which refer to expression levels are well known in the art, and "COL17A1^{high}" refers to a high level of COL17A1 expression relative to the population of cells being analyzed. In the context of the present disclosure, "high" or "hi" may be considered to represent an expression level within the top 50%, for example, within 3%, 5%, 10%, 15%, 20%, 30%, or 40%, of the target cell population. The cells in the cell composition of the present invention may be, for example, epidermal keratinocytes or mucosal epithelial keratinocytes, but other cells may also be included. The cells in the cell composition of the present invention can be, for example, cells in which at least 50%, 60%, 70%, 80%, 90%, 95%, 98%, or 99% of the cells express COL17A1. The cell composition of the present invention can contain at least 1 × 10³, 1 × 10⁴, 1 × 10⁵, 1 × 10⁶, 1 × 10⁷, or 1 × 10⁸ cells.

The cell composition of the present invention can be a cell composition for use in transplantation. The cell composition of the present invention can be a cell composition for use in the treatment of a disease or an injury of the skin. The cells in the cell composition of the present invention can be cells derived from stem cells. The cells in the cell composition of the present invention can be cells in which the expression of COL17A1 has been induced or maintained using a substance that induces or maintains the expression of COL17A1 in a cell. The substance that induces or maintains the expression of COL17A1 in a cell may be at least one of a ROCK inhibitor, an NADPH oxidase inhibitor, an iNOS inhibitor, and a COX inhibitor. The substance that induces or maintains the expression of COL17A1 in a cell may be Y27632 or apocynin.

The cells in the cell composition of the present invention may be frozen. The cell composition may be contained in a single container.

### Cell sheet

One of the embodiments of the present invention relates to a cell sheet comprising cells expressing COL17A1. Such a cell sheet can be produced by isolating and culturing cells expressing COL17A1. Such a cell sheet can be transplanted into a patient for use in treating the patient's disease or injury. The cell sheet can be for autologous or autologous transplantation.

Hereininbelow, the present invention will be specifically described based on the examples, but the present invention is not limited to these examples.

### Examples

### Example 1: Aging-associated decrease in the COL17A1 protein expression in the skin

The skin of the tail of mice differs from the skin of the back, and as observed in histological analyses of the stratified epidermal cell layer and epidermal melanocytes of human epidermis, the tail skin of aged mice is also characterized by atrophy with a reduction in the number of spinous cell layers in the scaly epidermis. The basal layer cells of young mice have a rectangular, longitudinal shape relative to the basement membrane, whereas aged basal layer cells have a more rounded or flattened morphology. In aged skin, there is a significant decrease in the total number of basal layer cells as well as MCM2⁺ non-dormant basal cells, indicating that stem cells in the epidermis and their cell division are reduced with age. Ultrastructural analyses of the epidermal basal layer cells and the epidermal-dermal junction using transmission electron microscopy (TEM) revealed that the number of electron-dense regions (hemidesmosome; HD) that anchor the basal cell layer to the basal plate significantly decreases with age. In addition, cell detachment from the basal layer cells is frequently observed in the transparent zone (lamina lucida; LL) of aged caudal skin, which is associated with the aging-induced decrease of HD from the basal cell layer.

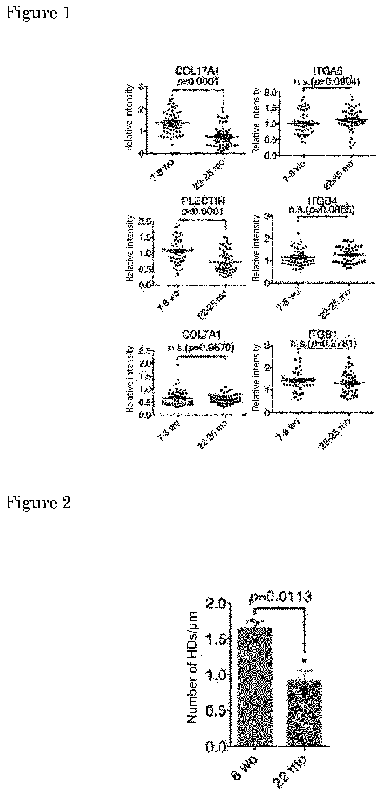

Therefore, the present inventors hypothesized that the HD components are directly involved in the process of skin aging. To test this, the present inventors analyzed the expression of various HD components and integrin β1 (ITGB1), a major cell adhesion molecule, in the caudal epidermis of young and old (weeks) mice. Results showed that the immunostaining level of COL17A1 is significantly decreased with aging (Fig. 1). Consistent with this, the immunoelectron microscopy analysis also revealed that the signal of COL17A1 in LL at the dermal-epidermal junction of aged skin is significantly reduced. The whole-body immunostaining also revealed a heterogeneous distribution of COL17A1 and ITGA6 in aged skin. In particular, the COL17A1-negative cell regions expressing ITGA6 were found in addition to the double-negative cell regions in the aged caudal epidermis. This indicates that the COL17A1 protein expression is the most unstable, and consequently, insufficiency of COL17A1 will eventually destabilize other HD components along with aging.

To investigate the role of COL17A1 in the stability of HD components, the present inventors analyzed the number of HDs in young and old mouse skin (Fig. 2). Furthermore, the protein degradation of COL17A1 is induced by persistent DNA damage responses such as X-ray irradiation in mice and genomic instability in TTD progeria mice. Similarly, ultraviolet (UV) irradiation also shows downregulation of the COL17A1 expression in several basal layer keratinocytes *in vitro* and *in vivo.* As a consequence, UV irradiation of the skin caused HD destabilization. Together, these data suggest that COL17A1 functions as a "stem cell checkpoint molecule", a stress/compatibility sensor that monitors the DNA damage response and determines whether stem cells will self-renew or differentiate. This stress-sensing mechanism can cause differences in the self-renewal ability of epidermal stem cell clones with different COL17A1 expression levels in the aged epidermal basal layer.

### Example 2: Clonal expansion of COL17A1⁺ epidermal stem cells during skin aging

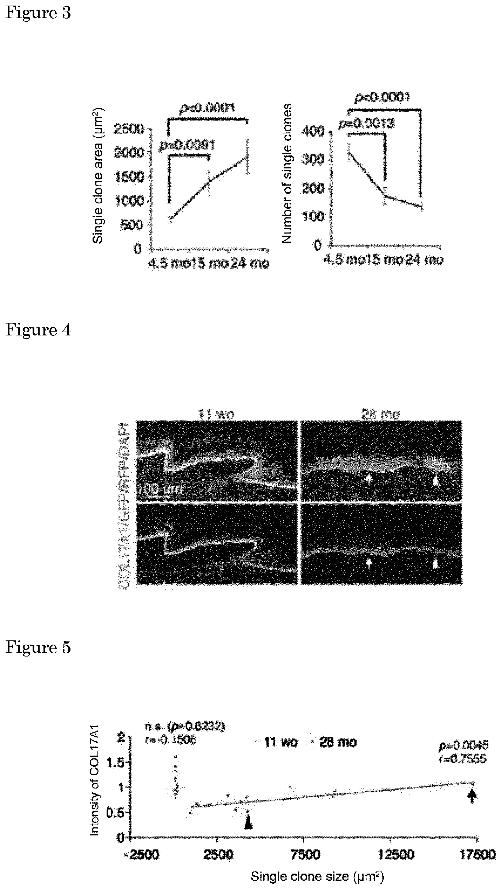

To visualize the dynamics of epidermal stem cell clones that emerge with aging and to track the fate of COL17A1-deficient clones, the present inventors generated a basal layer keratinocyte lineage tracing system with drug-induced multicolor labeling using K14-CreERT2; R26R Brainbow 2.1 mice. This system allows for stochastic multicolor labeling of basal stem/progenitor cells by Cre-LoxP-mediated recombination. After tamoxifen (TAM) treatment, GFP-, RFP-, YFP- or CFP-expressing cells were stochastically found in young (4.5 months) tail skin. In contrast, areas of single-colored keratinocyte clones increased significantly with age, accompanied by a decrease in the total number of clones (Fig. 3). This indicates that some stem cells undergo clonal proliferation at the expense of many other cells during physiological aging.

Next, to test the possibility that basal cell heterogeneity with different expression levels of COL17A1 may induce stem cell competition in the basal layer during aging, the present inventors examined the expression levels of COL17A1 by multicolor-labeled stem cell clones in the caudal interfollicular epidermis (IFE) of young (11-month-old) and old (28-month-old) mice. As shown in Figure 4, the COL17A1 expression in the monochromatic basal layer cells representing stem cell clones was generally decreased in aged skin, whereas the COL17A1 expression in the basal layer cells of young mice showed different levels of COL17A1 expression in each clone and generally decreased with aging. A detailed comparison of the clone size of basal layer keratinocytes and the COL17A1 expression levels revealed that the clone size of basal layer cells was positively correlated with the expression level of COL17A1 in aged mouse skin (Fig. 5). This indicates that stem cell clones that maintain higher levels of COL17A1 expression exhibit higher clonogenicity and become dominant in the epidermal basal layer during the physiological aging process. Furthermore, there was no significant induction of apoptotic cell death or so-called "aging" cells in the basal layer cells of aged skin. These data suggest that COL17A1^{high} epidermal stem cells compete with COL17A1^{low} cells to withstand epidermal aging.

### Example 3: Differential expression of COL17A1 causes competition of epidermal stem cells

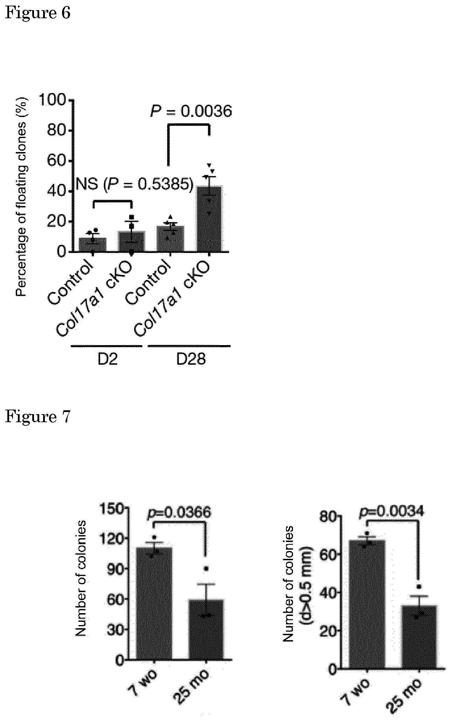

To investigate whether the appearance of COL17A1^{low/-} clones directly causes cell competition with surrounding neighbor COL17A1^{high} clones *in vivo,* the present inventors generated drug-induced Col17a1 gene knockout (cKO) mice by combining with a multicolor labeling system specific for basal layer keratinocytes. Rare induction of the loss of Col17a1 using a low-dose TAM administration resulted in monochromatically labeled Col17a1-deficient keratinocyte clones in the tail epidermis on day 2 after knockout induction. In the control mice, these clones increased in size at day 14 and then formed monochromatic columns (basal clones) at day 28. In contrast, many Col17a1-deficient basal layer keratinocyte clones, surrounded by unlabeled basal layer keratinocyte clones with high levels of COL17A1 expression, detached around day 14 and became loser (loser) cell clones (floating clones) by day 28 (Fig. 6). Consistent with this, the whole-mount analysis of the mouse tail revealed a marked exclusion of Col17a1-deficient cell clones from the skin. These data indicate that the COL17A1-expressing epidermal stem cells compete with the COL17A1-deficient epidermal stem cells in the niche, and that COL17A1 mediates skin homeostasis and the aging process through a cell competition mechanism in the epidermis.

To further investigate whether differential expression of COL17A1 directly mediates cell competition in the epidermis, the present inventors established a novel *in vitro* cell competition assay system by expressing short hairpin (sh)RNA in 3D cultured HaCaT human keratinocytes. EmGFP⁺COL17A1⁻ stable HaCaT cells expressing shCOL17A1 and the control EmGFP⁺COL17A1⁺ HaCaT cells expressing shScramble were generated and cultured in 3D culture system. shScramble-expressing HaCaT cells and shCOL17A1-expressing HaCaT cells neither showed obvious differences in the structure, stratification, number of proliferation, or apoptotic cells in 3D cultured epidermis. In contrast, co-culture of COL17A1⁻EmGFP⁺ cells and wild-type (COL17A1⁺) cells at a ratio of 1:10 significantly eliminated shCOL17A1-expressing cells. However, significant exclusion of COL17A1-expressing cells was not detected at a ratio of 1:3. These data indicate that Col17a1-deficient basal layer cells are eliminated from the basal layer as loser cells by a sufficient number of surrounding wild-type cells. In summary, these data suggest that COL 17A1-mediated cell competition occurs in the physiological aging process of human epidermis, and that COL17A1 expressed by epidermal stem cells confers neighboring epidermal stem cell populations with competitive self-renewal ability.

### Example 4: Maintenance of epidermal homeostasis by COL17A1-mediated SCD

To understand the COL17A1-mediated cell competition mechanism, the present inventors hypothesized that COL17A1 promotes SCD, which theoretically maintains clonal proliferation of COL17A1⁺ epidermal stem cells. To test this hypothesis, the present inventors analyzed the mitotic axis of basal layer cells *in vivo.* While normal basal lamina cells mostly showed cell division parallel to the basement membrane, Col17a1-deficient basal lamina keratinocytes abnormally induced perpendicular cell division, and generated basal lamina cells and an offspring layer located above the basal lamina. This indicates that COL17A1 mediates SCD, suggesting that COL17A1-mediated SCD pushes Col17a1-deficient basal layer cells with lost or reduced HD to separate from the basement membrane. Indeed, Col17a1-deficient keratinocytes eventually detach from the basal layer IFE.

Thus, these data indicate that COL17A1-dependent SCD, which promotes clonal proliferation of COL17A1⁺ winner (winner) cells, causes cell competition during the aging process. Importantly, the basal layer cells of the aging epidermis had an increased percentage of vertical cell division and often reduced expression levels of COL17A1. This ultimately leads to epidermal atrophy (thinning) due to impaired cell division accompanied by loss of the COL17A1 expression in areas of aged skin. In stark contrast, epidermal basal layer cells from the COL17A1 transgenic (tg) mice, in which basal layer cells maintain the COL17A1 expression, maintained cell division in a horizontal direction to the basement membrane. These data indicate that COL17A1-mediated SCD generates a mechanical driving force for cell competition, and withstands epidermal aging via competitive self-renewal events that result from cell spreading in the horizontal direction and occupation of the basement membrane region in adult epidermis.

In addition, there is a known phenomenon that the amplification of specific stem cell clones predominates in hematopoietic stem cells, and other stem cell clones disappear, and it is known that self-renewal of stem cells occurs competitively (competitive self-renewal). The molecules involved in this are also being identified. However, the actual state of "competitive self-renewal" under physiological conditions in the epidermis is not known, and the molecules responsible for this have not been clarified.

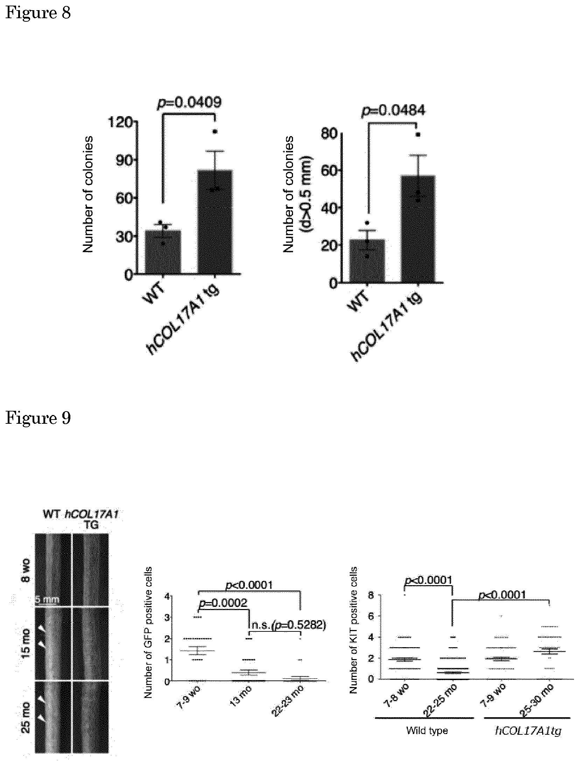

To test whether COL17A1-mediated SCD maintains clonal proliferation of epidermal stem cell clones, the present inventors used a colony formation assay, which is an *in vitro* assay said to be the gold standard for assessing the self-renewal ability of epidermal stem cells, including cells in the tail epidermis. Aged epidermal keratinocytes expressing low levels of COL17A1 had a reduced colony-forming capacity and generated smaller colonies compared to keratinocytes from younger tails (Fig. 7). In contrast, forced expression of hCOL17A1 in basal layer keratinocytes of mice promoted the colony-forming capacity of epidermal stem cells, with significantly larger colony size and number than in control mice (Fig. 8).

These data indicate that COL17A1 mediates clonal proliferation through its ability to proliferate epidermal stem cells. Consistent with this, the expression of COL17A1 in tg mice significantly rescued age-related epidermal thinning and age-related detachment. In summary, the COL17A1-dependent proliferative ability of epidermal stem cells to continuously undergo symmetric cell division (SCD) in colony assays explains the higher compatibility of COL17A1^{high} stem cell clones in epidermal homeostasis and cell competition during aging, and their ability to self-renew competitively.

### Example 5: Maintenance of a heterotypic cell lineage by COL17A1-expressing epidermal stem cells

Skin aging is characterized not only by epidermal aging, but also by melanocyte-related pigmentation abnormalities and dermal changes. To study the functional significance of epidermal stem cell aging in skin aging, the present inventors assessed whether physiological aging induces epidermal pigmentation abnormalities in the tail epidermis. It was observed that physiological aging gradually induced skin pigmentation abnormalities (heterogeneous skin pigmentation with areas of low pigmentation and high pigmentation), and ultimately induced hypopigmentation in the tail skin. Detailed microscopic examination of the wholemounts of the tail revealed that the pigmentation pattern became more heterogeneous with age. To visualize the distribution of melanocytic lineage cells in the epidermis, the present inventors analyzed the tail skin of Dopachrome tautomerase (Dct) promoter-regulated histone-H2B GFP (Dct-H2B GFP) tg mice. Section and whole-mount analyses showed that H2B GFP⁺ epidermal melanoblasts/melanocytes were observed in the tail of young mice, but these cells disappeared in an age-dependent manner in the epidermis of the aged tail. Consistent with this, microarray analysis of young and aged total epidermal cells ranked melanocyte genes as the top major change during aging. Importantly, highly pigmented melanocytes were observed in the basal lamina region of the 12-month-old tail, suggesting that these cells are occasionally present in the upper basal lamina region, where they are detached from the basal lamina and eliminated from the skin.

Next, to investigate whether changes in the epidermal melanocytes are induced by stem cell aging of epidermal keratinocytes, the present inventors examined whether loss of the HD components due to Col17a1 and/or Itga6 deficiency in the epidermal basal layer cells induces early skin pigmentation abnormalities. Longitudinal analysis revealed that Col17a1 deficiency induced relatively mild pigmentation abnormalities by inducing HD instability, and that the Col17a1 and Itga6 genes synergistically induced pronounced pigmentation abnormalities. Indeed, epidermal melanocytes tagged with Dct-H2B GFP were depleted from the skin when the Col17a1 or Itga6 genes were deleted.

Chronic UVB irradiation induces abnormal skin pigmentation and HD damage in mouse and human epidermis. The present inventors next examined whether chronic UVB exposure induces skin pigmentation abnormalities and HD damage, as well as their associated phenotypes in the caudal epidermis (Fig. 9). Following skin hyperpigmentation induced by repeated UVB irradiation, epidermal pigmentation abnormalities were induced within one month after the last UVB irradiation, similar to the Col17a1 or ITGA6 cKO mice. Ultrastructural analysis using TEM and immuno-TEM revealed that UVB irradiation inhibited the formation of mature HD and reduced the signal of COL17A1, similar to Col17a1 cKO.

Together, these data indicate that chronic UVB-mediated HD damage induces epidermal pigmentation abnormalities by impairing COL17A1-expressing epidermal keratinocytes. Furthermore, overexpression of COL17A1 in mouse basal layer keratinocytes significantly rescued the macroscopic and microscopic pigmentation abnormality phenotypes, as well as the age-related decrease in KIT⁺ epidermal melanocytes (Figure 9). In summary, these data indicate that during aging and photoaging, melanocytes in the epidermis are maintained by adjacent COL17A1-expressing epidermal stem cells in the epidermis.

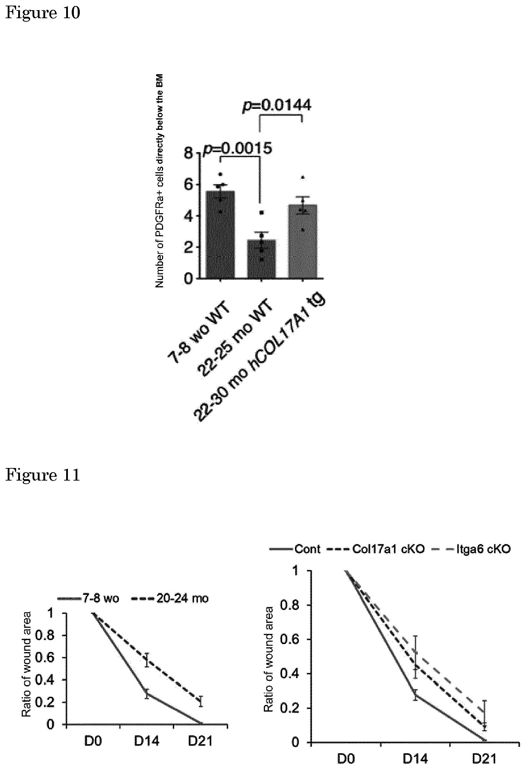

The regenerative ability of the skin decreases with age. The present inventors tested how age-related changes in epidermal stem cells are involved in skin wound healing, which proceed via epidermal stem cell cloning, and whether they affect dermal fibroblasts (Fig. 10). The analysis of PDGFRα-positive mesenchymal cells showed that aging significantly reduces the population located just below the epidermis, and that the forced expression of hCOL17A1 rescued the age-related loss of PDGFRα-positive cells. These data suggest that the COL 17A1-mediated maintenance of epidermal stem cells strengthens the stability of the epidermal-dermal junction and resists aging of the skin organ, including skin atrophy, fine wrinkles, and skin aging, induced by the failure.

### Example 6: HD is the key to skin organ regeneration

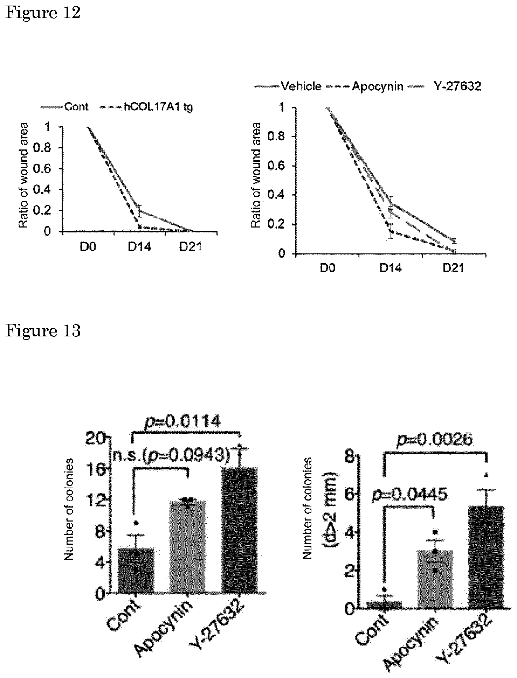

To test the above hypothesis, the present inventors performed a full-layer wound healing experiment using the tail skin of aged wild-type mice. Measurements of the wound area showed that physiological aging significantly delayed the wound healing process. The present inventors then analyzed the Col17a1 or ITGA6 deficient skin and found that both showed significantly delayed wound repair (Fig. 11), indicating that HD instability leads to poor wound healing in physiological aging. Forced expression of hCOL17A1 by basal layer keratinocytes in mice also promoted wound healing (Fig. 12). These data indicate that the COL17A1-mediated clonal expansion of epidermal stem cells is essential not only for stem cell competition for skin homeostasis, but also for regeneration of the full-layer skin wound healing.

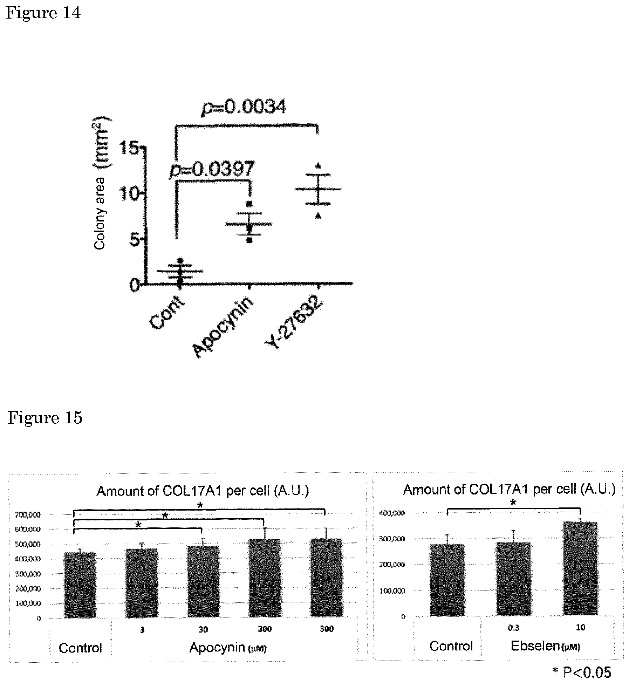

Since overexpression of COL17A1 promotes skin wound healing, the present inventors searched for novel compounds that induce the COL17A1 expression in keratinocytes *in vitro.* As shown in Table 1 and Fig. 15, the present inventors identified two chemicals that induce the COL17A1 expression in cultured keratinocytes, Y27632 and apocynin (Apocynin). To confirm *in vitro* that the effects of these compounds are mediated by an increase in the competitive self-renewal ability of epidermal stem cells exhibiting SCD, the present inventors performed a colony formation assay using human epidermal keratinocytes. Consistent with this, the colony number was significantly increased by Y-27632, and the colony size was significantly increased by both agents (Table 1, Fig. 13 and Fig. 14).

To confirm the beneficial effects *in vivo,* these drugs were administered to full thickness wounds in the skin of rodent tails. Both drugs significantly accelerated the wound repair process, similar to tg mice overexpressing human COL17A1 (Fig. 12). These results indicate that the COL17A1-inducible drugs promote skin wound healing through the promotion of re-epithelialization of the wound margins by proliferation of epidermal stem cells, and these findings open up a new avenue for new drugs to promote skin regeneration.

### Example 7: Search for substances that maintain and promote the competitive self-renewal ability of epidermal stem cells

The present inventors further searched for substances that induce or maintain the expression of COL17A1. Human epidermal keratinocytes were seeded in 384-well plates, cultured for 48 hours after addition of reagents, immunostained with an anti-COL17A1 antibody and a fluorescently labeled secondary antibody, and then the fluorescence intensity per cell was detected using a high content imaging system (HCS: High Content Screening). In addition to cell counting with DAPI, the expression level of COL17A1 was quantified.

As a result, the present inventors identified a group of substances that maintain the self-renewal ability of epidermal stem cells through the expression of COL17A1. However, it is difficult to distinguish them from substances that transiently increase the expression of COL17A1 but weaken the self-renewal ability in the long term, such as steroids and other cytotoxic substances. Therefore, it is not possible to exclude them by measuring the COL17A1 expression. By combining it with the colony formation assay, the present inventors succeeded in selecting substances that promote the COL17A1-mediated competitive self-renewal in the long term. The results are shown in Fig. 15 and Tables 1 and 2.

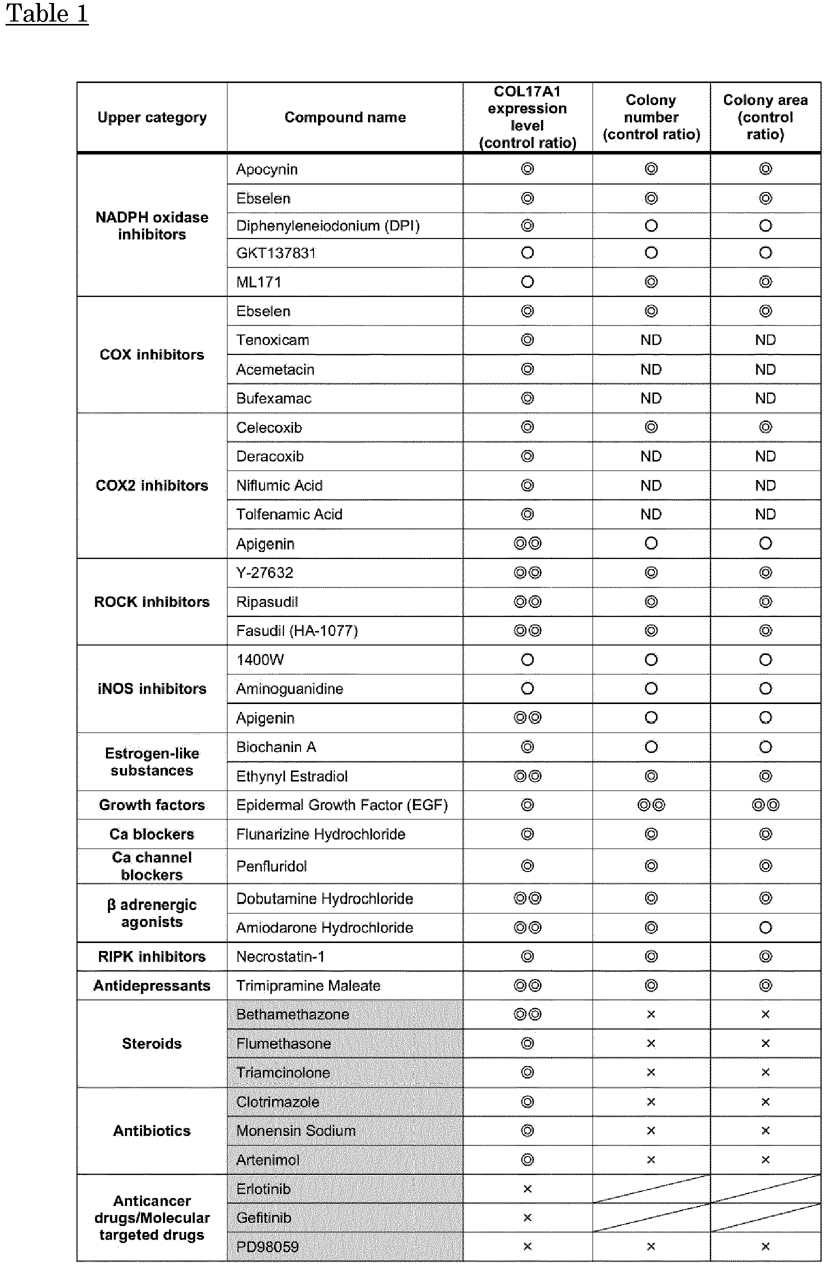

In Table 1, two double circles (⊚⊚) in the COL17A1 expression level (control ratio) represent a 2.0-fold or greater increase in the expression level, one double circle (⊚) represents a 1.2-fold or greater increase, and one circle (0) represents a 1.0-1.2-fold maintenance. Two double circles (⊚⊚) in the control ratio of the colony number or colony area indicates an increase of 1.2-fold or more, one double circle (⊚) indicates maintenance of 1.0-1.2-fold, and X indicates less than 1.0 (same as below), whereas nd (not done) indicates that it has not been analyzed.

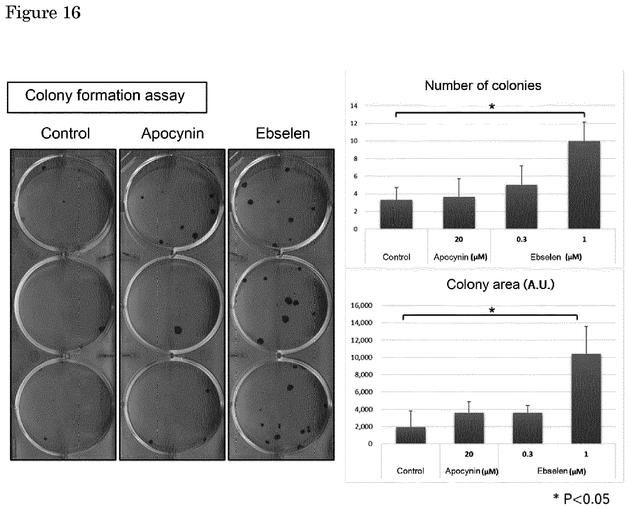

Among the substances that induce or maintain the expression of COL17A1 expressed by epidermal stem cells, the present inventors found that among them, many enhanced the colony-forming ability or self-renewal ability of epidermal stem cells (Fig. 16, Tables 1 and 2). On the other hand, there are some substances that were difficult to distinguish from stress-responsive changes in the COL17A1 expression and others that stably reduced the colony-forming ability. Therefore, by combining both the COL17A1 expression analysis and colony-forming ability, one can select substances with "competitive self-renewal ability" *in vivo.*

**Table 2**

| **Upper category** | **Compound name/ Extract name** | **COL17A1 expression level (control ratio)** | **Colony number (control ratio)** | **Colony area (control ratio)** |
|---|---|---|---|---|
| | Helenin | ⊚⊚ | ⊚ | ⊚ |
| | Lapachol | ⊚⊚ | ⊚ | ⊚ |
| | Apocynum Venetum leaf/stem extract | ⊚⊚ | ⊚ | ○ |
| | Morus Alba leaf extract | ⊚⊚ | ⊚ | ⊚ |
| | Gymnema Sylvestre leaf extract | ⊚ | ⊚ | ⊚ |
| | Camellia Sinensis leaf extract | ⊚ | ⊚ | ○ |
| | Eriobotrya Japonica leaf extract | ⊚ | ⊚ | ⊚ |
| | Silybum Marianum extract | ⊚⊚ | ⊚ | ○ |
| | Kaempferia Parviflora extract | ⊚⊚ | ⊚ | ⊚ |
| | Plantago Major seed extract | ⊚ | ⊚ | ⊚ |
| | Coffea Arabica (Coffee) seed extract | ⊚ | ⊚ | ⊚ |
| | Prunus Yedoensis leafextract | ⊚ | ○ | ○ |
| | Melia Azadirachta leaf extract | ⊚ | ⊚ | ⊚ |
| | Citrus Nobilis peel extract | ⊚ | ○ | ⊚ |
| | Lavandula Angustifolia extract | ⊚ | ○ | ○ |
| | Sophora Angustifolia root extract | ⊚ | ○ | ○ |
| | Oryza Sativa Rice bran extract | ⊚ | ○ | ○ |
| | Rosa Canina fruit extract | ⊚ | ○ | ○ |
| | Hamamelis Virginiana leaf extract | ⊚ | ○ | ○ |
| | Eucalyptus Globulus leaf extract | ⊚ | ○ | ○ |
| | Crataegus Cuneata Fruit extract | ⊚ | ○ | ○ |

**Table 3**

| | **Compound name** | **COL17A1 expression level (control ratio)** |
|---|---|---|
| **Anti-metabolites** | Tegafur | 0.90 |
| | Hydroxyurea | 0.92 |
| | Mercaptopurine | 0.83 |
| | Fluorouracil acetate | 0.88 |
| | Fludarabine phosphate | 0.90 |
| | Gemcitabine | 0.96 |
| **Alkylating agents** | Cyclophosphamide hydrate | 0.88 |
| | Ifosfamide | 0.84 |
| | Dacarbazine | 0.94 |
| | Carmustine | 0.94 |
| | Temozolomide | 0.98 |
| **Platinum drugs** | Cisplatin | 0.97 |
| **Antiestrogens** | Tamoxifen citrate | 0.78 |
| | Toremiphene citrate | 0.94 |
| **Aromatase inhibitors** | Exemestane | 0.93 |
| **Hormone regulators** | Testosterone propionate | 0.84 |
| | Mitotane | 0.98 |
| **Antibiotics** | Puromycin hydrochloride | 0.85 |
| **Immunosuppressants** | Cyclosporine | 0.97 |
| **Molecularly targeted drugs** | Erlotinib | 0.64 |
| | Gefitinib | 0.76 |
| | PD98059 | 0.84 |

Table 3 summarizes the anticancer drugs and molecularly targeted drugs that are found to decrease the expression of COL17A1. The expression of COL17A1 is altered by conventional chemotherapeutic agents such as antimetabolites and alkylating agents. In particular, the expression of COL17A1 is markedly reduced by molecularly targeted drugs that target cancer cells (Table 3).

### Example 8: Reduction of COL17A1 by anticancer drugs and various stresses

The anticancer agent hydroxyurea was administered to 7-week-old C57BL6 mice three times a month, and the tissues were collected three months later for immunostaining using an antibody for COL17A1. As a result, the expression of COL17A1 in the basal layer was markedly decreased in the hydroxyurea-treated mice (Fig. 17). In addition, HaCaT cells, which are human epidermal keratinocytes, were irradiated with radiation (20 Gy, 30 Gy) and UV light (20 mJ, 30 mJ) and the cells were collected after 24 and 72 hours, respectively, and Western blotting was performed using an antibody against COL17A1. The results showed a significant decrease in the amount of the COL17A1 protein under both radiation and UV irradiation. Furthermore, when HaCaT cells were treated with hydrogen peroxide, which induces oxidative stress, the expression of COL17A1 was analyzed in the same way, and a marked decrease was observed (Fig. 17).

These results indicate that various stresses such as anticancer drugs, ultraviolet light, radiation, and oxidative stress reduce the expression of COL17A1.

### Example 9: Experiment for improvement of skin damage caused by anticancer drugs as a result of COL17A1 overexpression

Transgenic mice overexpressing human COL17A1 (COL17A1-Tg) and control mice (Control) (about 18 months old) were acclimated to the experimental environment, then hair was removed from their back under anesthesia, and the mice were divided into the six groups below.
- Control mice without EGF receptor inhibitor (n = 3)
- COL17A1-Tg mice without EGF receptor inhibitor (n = 3)
- Control mice EGF receptor inhibitor (erlotinib) group (n = 4)
- COL17A1-Tg mice EGF receptor inhibitor (erlotinib) group (n = 4)
- Control mice EGF receptor inhibitor (gefitinib) group (n = 3)
- COL17A1-Tg mice EGF receptor inhibitor (gefitinib) group (n =3 )

Erlotinib or gefitinib, an EGF receptor molecular targeting agent, was dissolved in 0.5% methylcellulose solution at a concentration of 5 mg/ml and administered by intraperitoneal injection at a dose of 50 mg/kg/day daily from the start of the study. A 0.5% methylcellulose solution of the solvent was similarly administered to the control non-treated group. The transepidermal water evaporation rate (TEWL) was measured for each group of mice on day 7 after the start of erlotinib or gefitinib administration. Measurements were taken at specific sites on the back of the mice (two sites symmetrically located on the left side and right side from the center of the spine) using Tewameter TN300 (Courage + Khazaka, Integral Corporation, Germany).

Fig. 18 shows the test results. Fig. 18 shows that TEWL was significantly increased in the control mice treated with erlotinib or gefitinib, but in comparison, Tg mice overexpressing COL17A1 showed significantly lower TEWL values even under erlotinib or gefitinib treatment, suggesting that skin damage was reduced. This indicates that the skin damage induced by anticancer drugs is significantly improved by promoting the self-renewal ability of epidermal stem cells through overexpression of COL17A1.

### Example 10: Experiment for improvement of skin damage caused by anticancer drug administration with a substance that promotes the self-renewal ability of epidermal stem cells

Female C57BL6N mice (7 weeks old or 6 months old) were acclimatized to the experimental environment, and then hair was removed from their back under anesthesia. They were divided into the 6 groups (n = 4) below, so that their body weight values would be nearly uniform.
- 7 weeks old / no EGF receptor inhibitor (erlotinib) group
- 7 weeks old / EGF receptor inhibitor (erlotinib) + control solvent application group
- 7 weeks old / EGF receptor inhibitor (erlotinib) + 200 µM apocynin application group
- 6 months old / no EGF receptor inhibitor (erlotinib) group
- 6 months old / EGF receptor inhibitor (erlotinib) + control solvent application group
- 6 months old / EGF receptor inhibitor (erlotinib) + 200 µM apocynin application group

Erlotinib was dissolved in 0.5% methylcellulose solution at a concentration of 5 mg/ml and administered by intraperitoneal injection at a dose of 50 mg/kg/day daily from the start of the study. The control non-administered group received the 0.5% methylcellulose solution of the solvent in the same way. The control group applied with the solvent and the group applied with 200 µM apocynin received an aqueous solution of each preparation once a day, five times a week on the back. To the control solvent application group the solvent (50% ethanol/PBS) of apocynin was similarly applied. The TEWL of 7-week-old mice and 6-month-old mice were measured on day 7 and day 12, respectively, after the start of erlotinib administration. The measurement was performed at specific sites on the back of the mice (two sites symmetrically located on the left and right sides from the center of the spine) using Tewameter TN300 (Courage + Khazaka, Integral Corporation, Germany).

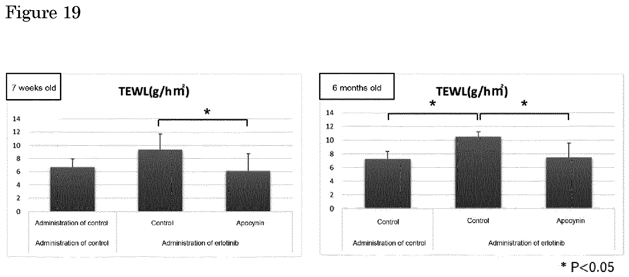

Fig. 19 shows the test results. The results in Fig. 19 show that TEWL was significantly increased in mice treated with erlotinib, but TEWL was significantly decreased and skin damage was reduced in the group treated with apocynin even under erlotinib administration. This indicates that the skin damage induced by the administration of a molecularly targeted drug can be significantly improved by a substance that promotes the cell competitive ability of epidermal stem cells.

### Example 11: Experiment for improvement of skin disorders in aged mice by a substance that promotes the self-renewal ability of epidermal stem cells

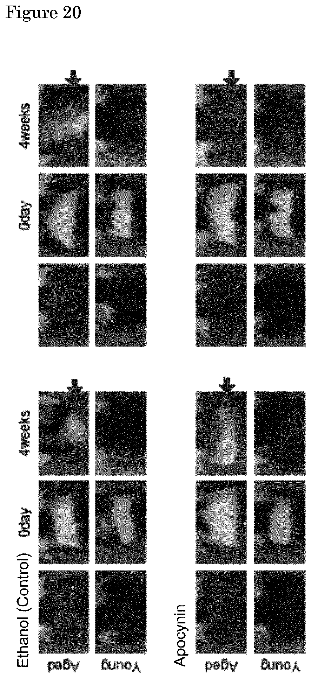

In the irritant skin disorder (dry eczema and alopecia) model induced by a high concentration of ethanol (100%) in the upper cervical-dorsal skin of aged mice, a substance (apocynin) that promotes the cell competitive ability of epidermal stem cells was continuously applied. As a result, as shown in Fig. 20, the skin damage was significantly reduced, and a significant preventive and therapeutic effect was obtained.

### Example 12: Experiment for improvement of the barrier function

The skin barrier function is a major function that the skin performs, and it prevents moisture from evaporating from the body and foreign substances from entering the body. However, when the epidermis thickens responsively to UVB irradiation, the barrier function deteriorates at the same time. In general, the stratum corneum water content and TEWL are used to evaluate the skin barrier function, and together with reactive epidermal thickness, they are considered to be indicators that reflect the degree of a skin disorder caused by UVB irradiation and anticancer drugs.

Hairless mice HOS: HR-1 females (7 weeks old) were acclimatized to the experimental environment and then grouped into the 6 groups below, so that the body weight values were nearly uniform.
- No ultraviolet irradiation group (n = 2)
- Ultraviolet (UVB) irradiation + solvent application group (n=4)
- Ultraviolet (UVB) irradiation + 200 nM apocynin application group (n = 4)
- Ultraviolet (UVB) irradiation + 200 µM apocynin application group (n = 4)
- Ultraviolet (UVB) irradiation + 2 µM Y-27632 application group (n = 4)
- Ultraviolet (UVB) irradiation + 20 µM Y-27632 application group (n = 4)

In the above solution application group, aqueous solutions of each preparation were applied to hairless mice on their back once a day for five days, starting from the day before ultraviolet irradiation. UVB irradiation was carried out using a Yayoi ultraviolet irradiation system (Y-UV-Lab-K-D5 lamp type) at an intensity of 150 mJ/cm² once a day. The stratum corneum water content and TEWL of the irradiated area were measured for each group of mice on the fourth day after the start of ultraviolet irradiation. The measurements were performed using Corneometer CM825 and Tewameter TN300 (Courage + Khazaka, Integral Corporation, Germany) at specific sites on the back of the mice. The measurements for each group of mice were performed at specific sites on the back of the mice (two sites symmetrically on the left side and right side from the center of the spine).

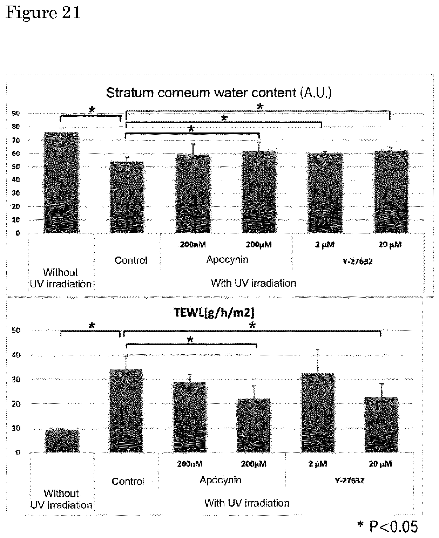

Fig. 21 shows the effect of substances that promote the self-renewal ability of epidermal stem cells on retention of the stratum corneum water content in response to ultraviolet irradiation. The water content of the stratum corneum after ultraviolet irradiation was significantly lower than that of the control group without ultraviolet irradiation, but the water content of the stratum corneum was significantly higher in the apocynin 200 µM group and the Y-27632 2 µM and 20 µM groups. This indicates that substances that promote the cell competitive ability of epidermal stem cells have the effect of preserving the stratum corneum water content in response to ultraviolet irradiation.

Furthermore, the present inventors analyzed the inhibitory effect of substances that promote the self-renewal ability of epidermal stem cells on TEWL in response to ultraviolet irradiation. As shown in Fig. 21, the TEWL after ultraviolet irradiation was significantly higher than that of the control group without ultraviolet irradiation, but the apocynin 200 µM administration group and Y-27632 20 µM administration group showed significantly lower TEWL. This indicates that substances that promote the cell competitive ability of epidermal stem cells have an inhibitory effect on the increase of TEWL caused by ultraviolet irradiation.

### Example 13: Evaluation of improvement on the skin elasticity (skin firmness)

Changes in the conditions of the dermis due to ultraviolet rays and aging cause the skin to lose its elasticity (= firmness), resulting in wrinkles and sagging. The elasticity of the skin is measured by the stretching of the skin when it is pulled and the return of the skin when it is released. An increase in skin elasticity may lead to an improvement in wrinkles, sagging or firmness of the skin.

Hairless mice HOS: HR-1 females (7 weeks old) were acclimatized to the experimental environment and then grouped into the three groups (n = 4) below, so that the body weight values would be nearly uniform.
- No UV irradiation group
- UV (UVA+UVB) irradiation + solvent application group
- Ultraviolet (UVA+UVB) irradiation + 200 µM apocynin application group

In the solution application group, aqueous solutions of each preparation were applied to the back three times a week for five weeks, once a day starting from the day before UV irradiation. In the "UV + solvent application group", the solvent of apocynin (50% ethanol/PBS) was applied in the same way. For UVB irradiation, a Yayoi ultraviolet irradiation system (Y-UV-Lab-K-D5 lamp type) was used, and the animals were irradiated with UVA at an intensity of 2 J/cm² and UVB at an intensity of 150 mJ/cm² three times a week for five weeks.

Skin elasticity was measured on the back (at two sites on the left side and right side symmetrically from the center of the spine) of each group at the fifth week after the start of UV irradiation. Skin elasticity was measured using Cutometer MPA580 (Courage + Khazaka, Integral Corporation, Germany) (parameter settings: 300 mba, 2 sec ON time, 2 sec OFF time), and the average value of three measurements was used for analysis. By suctioning the skin on the back of the mouse for 2 seconds and then releasing the suction 2 seconds later, the length of skin stretched at each time point immediately after suctioning, immediately before releasing the suction, and immediately after releasing the suction was measured; and they are rapid stretching in the initial stage of suctioning (Ue: unit mm), maximum skin stretching immediately before releasing the suction (Uf: unit mm), and rapid recovery (Ur: unit mm) immediately after suction release. R5=Ur/Ue was used as an index of net elasticity, and R7=Ur/Uf as an index of elasticity.

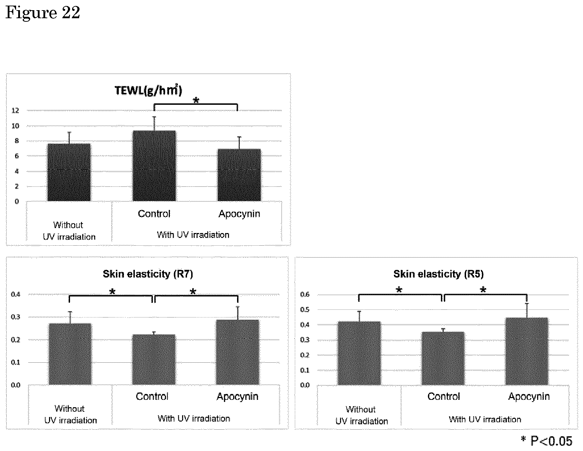

As shown in Fig. 22, UV irradiation significantly reduced the value of skin elasticity, but the test group that applied the substance that promotes the self-renewal ability of epidermal stem cells significantly improved the skin elasticity compared to the comparison group. The increase in skin elasticity may lead to an improvement in wrinkles, sagging or firmness of the skin. This suggests that the application of substances that promote the cell competitive ability of epidermal stem cells can improve the wrinkles, sagging, or firmness of the skin.

### Example 14: Evaluation of improvement in the skin elasticity (skin firmness) through long-term application

To investigate the effect of substances that promote the cell competitive ability of epidermal stem cells on skin elasticity, female hairless mice (HOS: HR-1) (7 weeks old) were acclimated to the experimental environment and then divided into the three groups (n = 2) below, so that their body weight values would be nearly uniform.
- Control (solvent) application group
- Apocynin 200pM application group
- Retinol 0.1% application group

The aqueous solution of each preparation was applied to the back once a day, three times a week for seven weeks. TEWL measurements and skin elasticity measurements were performed on the back of each group (at two sites on the left side and right side symmetrically from the center of the spine) 7 weeks after the start of application (day 53). TEWL measurements were performed at specific sites on the back of mice (two sites on the left side and right side symmetrically from the center of the spine). The elasticity of the skin was measured using Cutometer MPA580 (Courage + Khazaka, Integral Corporation, Germany) (parameter settings: 300 mba, 2 sec ON time, 2 sec OFF time), and the average value of the three measurements was used for analysis. By suctioning the skin on the back of the mouse for 2 seconds and then releasing the suction 2 seconds later, the length of the skin stretched was measured at each time point immediately after suctioning, immediately before suction release, and immediately after suction release; and they are return of the skin at the time of suction release (Ua: unit mm), rapid stretching in the initial stage of suctioning (Ue: unit mm), maximum degree of skin stretch just before suction release (Uf: unit mm), and the amount of rapid recovery immediately after suction release (Ur: unit mm). R5=Ur/Ue was set as the index of net elasticity, and R7=Ur/Uf as the index of elasticity. R2 = Ua/Uf was set as the index of gross elasticity.

As shown in Fig. 23, the test group that received long-term continuous application of the substance that promotes the self-renewal ability of epidermal stem cells significantly improved skin elasticity in all three indices compared to the retinol of comparison. Furthermore, retinol significantly increased the TEWL of the skin and suppressed the barrier function of the skin when continuously applied, but the normal barrier function was maintained for substances that promote the self-renewal ability of epidermal stem cells. Therefore, continuous application of substances that promote the self-renewal ability of epidermal stem cells may increase the elasticity of the skin while maintaining the normal barrier function of the skin, resulting in the prevention and improvement of wrinkles, sagging, and firmness of the skin.

### Example 15: Experiment for wrinkle improvement

In a mouse model in which wrinkle formation was induced by long-term exposure to ultraviolet (UVA) light, the present inventors examined the effect of substances that promote the self-renewal ability of epidermal stem cells on the improvement of wrinkles.

Hairless mice HOS: HR-1 females (7 weeks old) were acclimated to the experimental environment and then grouped into the three groups below, so that their body weight values would be nearly uniform.
- No UV irradiation group (n = 2)
- UV (UVA+UVB) irradiation + solvent application group (n = 4)
- Ultraviolet (UVA+UVB) irradiation + 200 µM apocynin application group (n =4)
- UV (UVA+UVB) irradiation + 0.1% retinol application group (n = 2)

In the above solution application group, aqueous solutions of each preparation were applied to hairless mice once a day starting from the day before UV irradiation, three times a week on the back for five weeks. In the "UV + solvent application group", the solvent of apocynin (50% ethanol/PBS) was applied in the same way. Retinol, which is known to be effective for wrinkle improvement, was applied in the same way at a concentration of 0.1% as a comparative control group. UVB irradiation was performed using a Yayoi ultraviolet irradiation system (Y-UV-Lab-K-D5 lamp type), with an intensity of 20 J/cm² of UVA and 100 mJ/cm² of UVB three times a week for four weeks.

For each group of mice from the first to the fourth week after the start of UV irradiation, the back was photographed with a high-resolution three-dimensional image analyzer PRIMOS-CR (Canfield Scientific, Inc., USA). Based on the images obtained, total wrinkle volume (mm³) and total wrinkle mean depth (µm) were measured with the Primos OMC3_22 software. Skin flexibility was measured using Cutometer MPA580 (Courage + Khazaka, Integral Corporation, Germany), and the value of R0 was used as skin flexibility. TEWL was measured on the back of each group (at two sites on the left side and right side symmetrically from the center of the spine) using Tewameter TN300 (Courage + Khazaka, Integral Corporation, Germany).

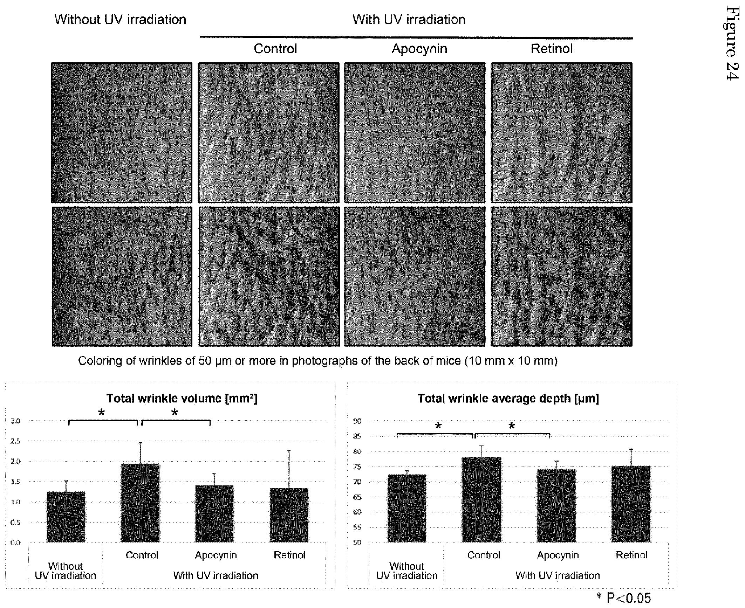

As shown in Fig. 24, UV irradiation significantly increased wrinkles in the skin, but the formation of wrinkles was suppressed in the test group to which the substance that promotes the cell competitive ability of epidermal stem cells was applied, compared to the control group; and the degree of suppression was more pronounced than that of retinol. The total volume of wrinkles (mm³) and the average depth of total wrinkles (µm) were measured. As shown in Fig. 24, UV irradiation significantly increased wrinkles in the skin, but the test group that applied the substance that promotes the cell-competition ability of epidermal stem cells improved significantly. This suggests that the application of substances that promote the self-renewal ability of epidermal stem cells can improve skin wrinkles.

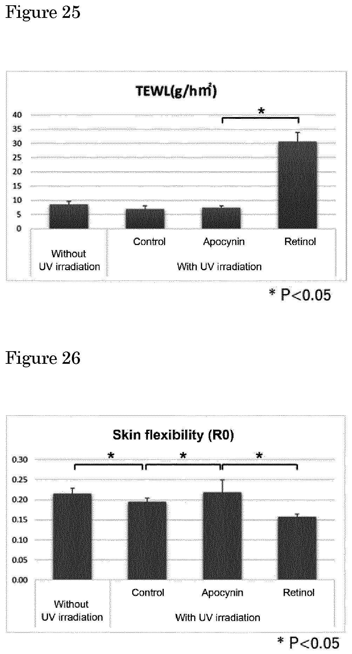

When transepidermal water evaporation (TEWL) was measured for each group of mice, there was a marked increase in TEWL in the retinol-applied group compared to the other groups (Fig. 25). On the other hand, the TEWL values in the group to which the substance that promotes the self-renewal ability of epidermal stem cells was applied did not differ from the control, suggesting that the substance reduces wrinkles while maintaining the normal barrier function.

Since there was a significant enhancement of TEWL in retinol, skin flexibility (R0), which is supposed to reflect the state of the stratum corneum, was measured for each group of mice. As a result, although UV irradiation significantly decreased the values of skin flexibility, the test group to which the substance promoting the self-renewal ability of epidermal stem cells was applied significantly improved the skin flexibility compared to the retinol of comparison (Fig. 26). This indicates that substances that promote the cell competitive ability of epidermal stem cells are more effective than retinol in improving wrinkles while maintaining a good stratum corneum condition.

### Example 16: Experiment for improvement of skin roughness (apocynin)

Using a skin irritation model induced by a 10% sodium lauryl sulfate (SDS) solution (a test that serves as a standard for chemical-induced skin damage), the present inventors administered a substance that promotes the competitive self-renewal ability of epidermal stem cells (apocynin) and evaluated the effect of the skin roughness-preventing or -ameliorating agent in improving skin roughness.

After acclimation of hairless mice (HOS: HR-1) to the experimental environment, the mice were divided into the three groups below, so that the body weight values were nearly uniform.
- SDS non-treated group (n = 2)
- SDS treatment + solvent application group (n = 4)
- SDS treatment + 200 µM apocynin application group (n = 4)

On the back of hairless mice, a cotton ball soaked in 500 µL of 10% SDS solution (dissolved in 70% ethanol) was applied for 30 min on days 1, 2, 3, and 4. Apocynin was applied to the back every day from the day before the experiment until day 4. For each group of mice on the third day after the start of the experiment, TEWL was measured at specific sites on the back (two sites on the left side and right side symmetrically from the center of the spine) as described above. On the eighth day, the images were taken by PRIMOS-CR (Canfield Scientific, Inc., Integral Corporation), a high-resolution 3D image analysis system. Based on the obtained images, for the two sites (10 mm × 10 mm each) on the left side and right side symmetrically from the center of the spine, same as the sites of TEWL measurement, the OMC3_22 software was used to measure the degree of skin roughness in terms of Ra (arithmetic mean roughness) and Rz (ten-point average roughness), which are parameters representing the surface roughness of the skin in the height direction.

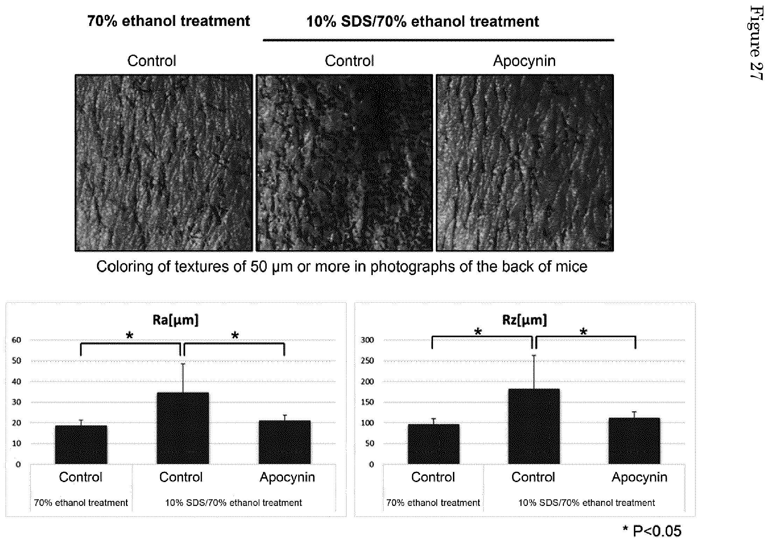

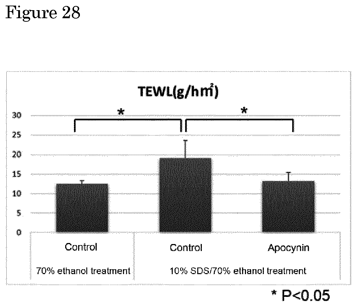

From the test results shown in Figures 27 and 28, in the SDS-treated control, TEWL was significantly increased and the Ra and Rz values, which are indicators of skin roughness, were increased; and there was a significant induction of skin roughness. On the other hand, mice treated with a substance that promotes the self-renewal ability of epidermal stem cells (apocynin) showed significantly lower TEWL on the back and significantly reduced Ra and Rz values on the surface of the back compared to the solvent-applied control group.

These results show that a substance that promotes the self-renewal ability of epidermal stem cells (apocynin) has a remarkable effect in preventing or improving skin roughness.

### Example 17: Experiment for improvement of skin roughness (ebselen)

Using a skin irritation model induced by 10% sodium lauryl sulfate (SDS) solution, the present inventors administered a substance that promotes the competitive self-renewal ability of epidermal stem cells (ebselen) and evaluated the effect of the skin roughness-preventing or -ameliorating agent in improving skin roughness.

On the back of hairless mice, a cotton ball soaked in 500 µL of 10% SDS solution (dissolved in 70% ethanol) was applied for 30 min on days 1, 2, 3, and 4. Ebselen was applied to the back every day from the day before the experiment until day 4. For each group of mice on the eighth day after the start of the experiment, TEWL was measured at specific sites on the back (two sites on the left side and right side symmetrically from the center of the spine) as described above. At the same time, the images were taken by PRIMOS-CR (Canfield Scientific, Inc., Integral Corporation), a high-resolution 3D image analysis system. Based on the obtained images, for the two sites (10 mm × 10 mm each) on the left side and right side symmetrically from the center of the spine, same as the sites of TEWL measurement, the OMC3_22 software was used to measure the degree of skin roughness in terms of Ra (arithmetic mean roughness) and Rz (ten-point average roughness), which are parameters representing the surface roughness of the skin in the height direction.

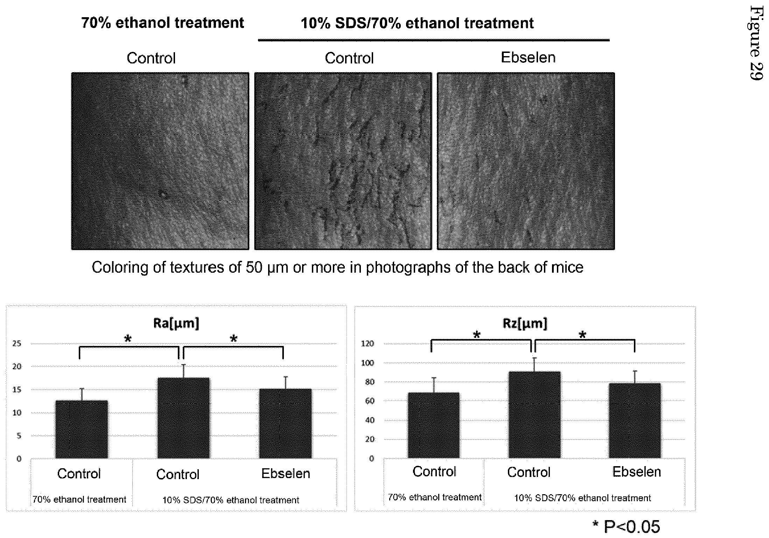

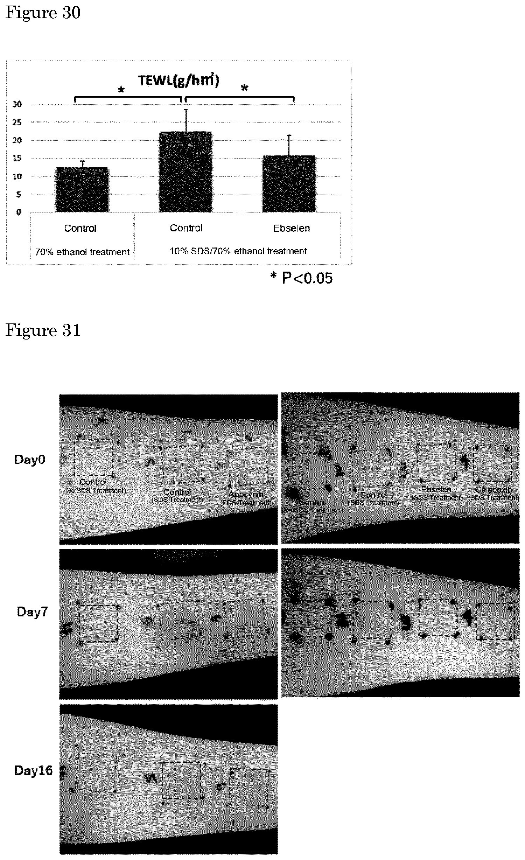

From the experiment results shown in Figures 29 and 30, in the SDS-treated control, TEWL was significantly increased and the Ra and Rz values, which are indicators of skin roughness, were increased; and there was a significant induction of skin roughness. On the other hand, mice treated with a substance that promotes the self-renewal ability of epidermal stem cells (ebselen) showed significantly lower TEWL on the back and significantly reduced Ra and Rz values on the surface of the back compared to the solvent-applied control group.

These results show that a substance that promotes the self-renewal ability of epidermal stem cells (ebselen) has a remarkable effect in preventing or improving skin roughness.

### Example 18: Experiment for improvement of human skin roughness

Two subjects were administered with substances that promote the self-renewal ability of epidermal stem cells (apocynin, ebselen, and celecoxib) using a test of skin roughness induced by 10% sodium lauryl sulfate (SDS) solution (a test that serves as a standard for chemical-induced skin damage in humans), and the effect of the skin roughness-preventing or -ameliorating agent in improving skin roughness was evaluated.

Six 1.5-cm square areas were placed on the subject's medial forearm, and each was subjected to the treatments below.
- SDS non-treated (70% ethanol treatment) group
   SDS treatment + solvent (50% ethanol/PBS) application group
- SDS treatment + apocynin (200 pM) solution application group
- SDS treatment + ebselen (0.25%) solution application group
- SDS treatment + celecoxib (2%) solution application group

A cotton ball permeated with 500 µL of 10% SDS solution (dissolved in 70% ethanol) was applied to the test area for 30 minutes each on day 0 and day 1. Apocynin was applied on day 0 before SDS treatment, applied after treatment, and then applied to the test area once daily until day 9. The TEWL at each test area was measured before and after SDS treatment, and daily from day 1 to day 7. For each area, the TEWL value before SDS treatment was subtracted from the TEWL value on the measurement day, and this value was defined as "ΔTEWL", and this value was used for evaluation. The test area was photographed with a digital camera and with PRIMOS-CR (Canfield Scientific, Inc., USA, Integral Corporation), a high-resolution three-dimensional image analyzer. For the PRIMOS images, the Primos OMC3_22 software was used to measure the degree of skin roughness in terms of "Ry (maximum height)" and "Rz (ten-point average roughness)", which are parameters representing surface roughness in the height direction. For each site, the value obtained by subtracting the Rz or Ry value of the SDS non-treated control from the Rz or Ry value on the measurement day was defined as "ΔRz" and "ΔRy", and these values were used for evaluation.

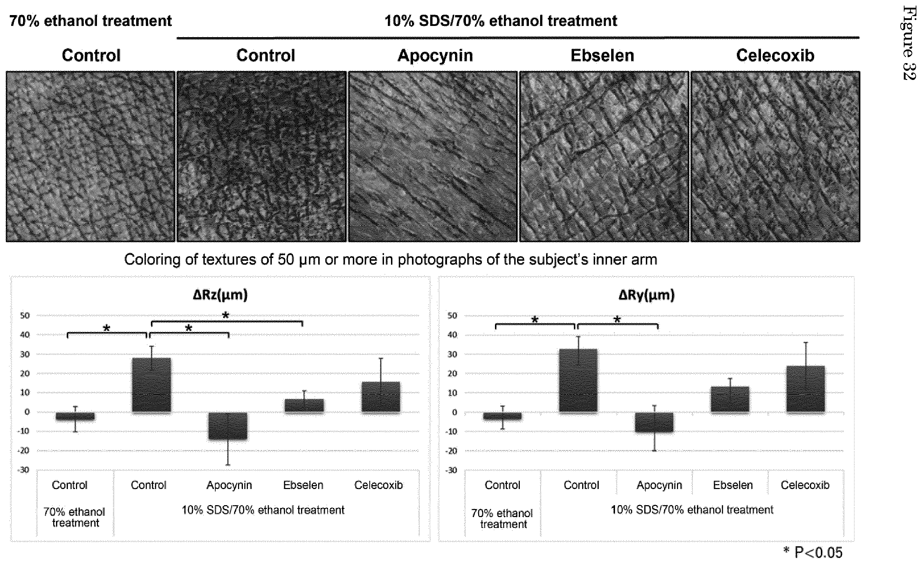

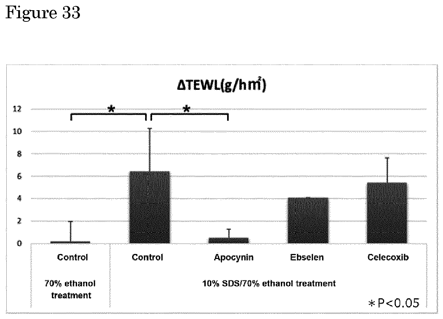

Figures 31-33 show the test results. As shown in Fig. 31, after SDS treatment, redness and skin irritation were clearly observed in the affected areas applied with the control and each solution. Furthermore, on the 16th day, pigmentation was observed in the affected area of the control, but almost no pigmentation was observed in the area where apocynin was applied. Further, as shown in Fig. 32, the value of ΔTEWL increased significantly due to the skin roughness induced by SDS, but the application of apocynin, ebselen or celecoxib significantly decreased the value of ΔTEWL. Furthermore, as seen in the results of Fig. 33, analysis of skin texture based on high-resolution photographs of the skin surface shows that the SDS-treated control significantly induced skin roughness, but it was significantly reduced by the application of apocynin, ebselen, or celecoxib. Furthermore, the ΔRz and ΔRy values, which indicate the roughness of the skin surface and are used as an indicator of skin roughness, significantly increased in the SDS-treated control, while these ΔRz and ΔRy values significantly decreased in the affected area after application of apocynin, ebselen, or celecoxib. This indicates that the skin roughness and pigmentation induced by the SDS solution are significantly improved by substances that promote the competitive self-renewal ability of epidermal stem cells.

From the results shown in Figures 31-33, the substances that promote the competitive self-renewal ability of epidermal stem cells have a remarkable effect on preventing or improving skin roughness in humans, and are considered to be effective in preventing and treating skin disorders caused by chemical substances.

### Manufacturing Example 1

### (1) Production of Apocynum Venetum (Apocynum venetum) Leaf/Stem Extract

The Apocynum Venetum Leaf/Stem Extract was made by Tokiwa Phytochemical Co. Ltd. The dried leaves of Apocynum venetum were crushed, 60% ethanol was added to the crushed material, and after cyclic heating, the extract was filtered to obtain the extract. The first and second extracts were combined, concentrated under reduced pressure and adjusted to pH 3, and the mixture was stirred overnight. The filtrate was filtered to remove insoluble material, and the resulting filtrate was passed through a synthetic adsorption resin, washed with water, and the fraction desorbed with 70% ethanol was collected, concentrated under reduced pressure, and spray dried to obtain the Apocynum Venetum Leaf/Stem Extract.

### (2) Mulberry (Morus alba) extract

The mulberry extract was produced by Tokiwa Phytochemical Co. Ltd. To the leaves of mulberry (Morus alba), 50% ethanol was added, and after heating and refluxing, the extract was obtained by filtration. After the extract was concentrated under reduced pressure, excipients were added, and the extract was dried to obtain the mulberry extract.

### (3) Production of gymnema (Gymnema sylvestre) extract

The gymnema extract was manufactured by Tokiwa Phytochemical Co. Ltd. Crushed gymnema (Gymnema sylvestre) leaves were extracted by heat reflux with 70% ethanol and filtered to obtain the extract. The obtained extract was concentrated under reduced pressure, and then dried under reduced pressure to obtain the gymnema extract.

### (4) Tea (Camellia sinensis) extract

The tea extract was made by Tokiwa Phytochemical Co. Ltd. The tea (Camellia sinensis) leaves were extracted with hot water and filtered to obtain the extract. The obtained extract was purified by an adsorption resin, then concentrated under reduced pressure and dried to obtain the tea extract.

### (5) Maria thistle (Silybum marianum) extract

The Maria thistle extract was manufactured by Tokiwa Phytochemical Co. Ltd. Maria thistle (Silybum marianum) fruits (defatted sludge) were extracted with 90-95% ethanol by heat reflux and filtered to obtain the extract. The extract was concentrated under reduced pressure and purified with ethanol, followed by concentration under reduced pressure and warm air drying to obtain the Maria thistle extract.

### (6) Loquat (Eriobotrya japonica) leaf extract

The loquat leaf extract was manufactured by Tokiwa Phytochemical Co. Ltd. Crushed loquat (Eriobotrya japonica) leaves were extracted with 80% ethanol by heat reflux and filtered to obtain the extract. After the decolorization process was carried out by adding activated carbon to the obtained extract, the decolorized solution was concentrated under reduced pressure and dried under reduced pressure to obtain the loquat leaf extract.

### (7) Production of black turmeric (Kaempferia parviflora) extract

The black turmeric extract was manufactured by Tokiwa Phytochemical Co. Ltd. The dried chips of black turmeric (Kaempferia parviflora) rhizome were crushed by a mixer, and 80% ethanol was added to the crushed material, and then the extract was obtained by filtration after heat reflux. The first and second extracts were combined, concentrated under reduced pressure, and then dried under reduced pressure with an excipient to obtain the black turmeric extract.

### Manufacturing Example 2

The plant extracts shown in Table 4 were extracted from the respective parts of various plants using the prescribed extraction solvents.

**Table 4**

| **Plant extract** | **Starting Material** | **Extraction solvent** |
|---|---|---|
| Absorage | Plantago Major Seed | 1,3-butylene glycol (BG) |
| Caffenoage | Coffea Arabica (Coffee) Seed | 1,3-butylene glycol |
| Sakura extract B | Prunus Yedoensis Leaf | 1,3-butylene glycol |
| Neem leaf liquid B | Melia Azadirachta Leaf | 1,3-butylene glycol |
| MandarinClear | Citrus Nobilis (Tangerine) Peel | 1,3-butylene glycol |
| ecofarm Lavender B | Lavandula Angustifolia (Lavender) Flower | 1,3-butylene glycol |
| Pharcolex Clara B | Sophora Angustifolia Root | 1,3-butylene glycol |
| Pharcolex rice nuka BK | Oryza Sativa (Rice) Bran | 1,3-butylene glycol |
| Pharcolex Novara B | Rosa Canina Fruit | 1,3-butylene glycol |
| Pharcolex Hamameris B | Hamamelis Virginiana (Witch Hazel) Leaf | 1,3-butylene glycol |
| Pharcolex Eucalyptus B | Eucalyptus Globulus Leaf | 1,3-butylene glycol |
| Pharcolex Hawthorn B | Crataegus Cuneata fruit | 1,3-butylene glycol |

The present specification shows the preferred embodiments of the present invention, and it is clear to those skilled in the art that such embodiments are provided simply for the purpose of exemplification. A skilled artisan may be able to make various transformations, and add modifications and substitutions without deviating from the present invention. It should be understood that the various alternative embodiments of invention described in the present specification may be used when practicing the present invention. Further, the contents described in all publications referred to in the present specification, including patents and patent application documents, should be construed as being incorporated the same as the contents clearly written in the present specification by their citation. The present application is an application claiming priority to JP 2019-59616 (filing date: March 27, 2019), which is incorporated herein by reference in its entirety.

### Industrial Applicability

As described above, the present inventors found that COL17A1 (type XVII collagen) is involved in skin aging and regeneration, as well as in skin damage caused by anticancer drugs and radiation, and identified compositions useful for promotion of skin wound healing, inhibition of skin aging, regulation of cell competition, and improvement of the regenerative ability of epidermal stem cells. The results of this study are as follows. Continuing treatment for a skin disorder caused by anticancer drugs has become a major challenge and a prognostic issue. The number of cancer patients who continue to work while continuing cancer treatment is increasing every year, and the need for appearance care is extremely high. The compositions, cells, and methods disclosed in the present application may be effectively used in the fields of continuation of anticancer drug treatment, surgical treatment, regenerative medicine, and aesthetics.

## Claims

1. A composition for use in promoting skin wound healing, or preventing or improving a skin ulcer or a bedsore, comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

2. A composition for use in inhibiting skin aging, comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

3. A composition for enhancing the regenerative ability of epidermal stem cells, comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

4. A composition for use in preventing or improving a skin disorder caused by an anticancer agent, comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

5. A composition for use in anti-wrinkle treatment, comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

6. The composition of claim 5, wherein the anti-wrinkle treatment is due to at least one of the following: improvement of skin elasticity, improvement of stratum corneum function, improvement of skin moisturizing ability, and improvement of skin barrier function.

7. A composition for use in anti-spot treatment, comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

8. A composition for use in improving rough skin, comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

9. The composition of any one of claims 1-8, wherein the substance that induces or maintains the expression of COL17A1 in a cell further maintains the self-renewal ability of epidermal stem cells.

10. The composition of any one of claims 1-9, wherein the substance that induces or maintains the expression of COL17A1 in a cell is selected from the group consisting of an NADPH oxidase inhibitor, a COX inhibitor, an iNOS inhibitor, a ROCK inhibitor, and an estrogen-like substance.

11. The composition of claim 10, wherein the NADPH oxidase inhibitor is selected from the group consisting of apocynin, ebselen, diphenyleneiodonium (DPI), GKT137831, AEBSF, GK-136901, ML171, Coenzyme Q10 (CoQ10), VAS2870, and VAS3947.

12. The composition of claim 10, wherein the COX inhibitor is selected from the group consisting of: celecoxib, deracoxib, tolfenamic acid, niflumic acid, FR122047, LM-1685, SC-791, BTB02472, Nimesulide, SPB04674, curcumin, diclofenac, 4'-hydroxydiclofenac, DuP-697, ebselen, ETYA, flubiprofen, ibuprofen, indomethacin, Meloxicam, NPPB, NS-398, pterostilbene, resveratrol, SC-560, SKF-86002, TXA (tranexamic acid), TXC (Cetyl Tranexamate HCl), Panax Ginseng extract, and sulphide sulindac.

13. The composition of claim 10, wherein the iNOS inhibitor is selected from the group consisting of 1400W, L-NIL, aminoguanidine, BYK190123, S-ethylisothiourea, S-methylisothiourea, S-aminoethylisothiourea, 2-iminopiperidine, butylamine, ONO-1714 ((1S,5S,6R,7R)-7-chloro-3-imino-5-methyl -2-azabicyclo[4.1.0]heptane hydrochloride), AMT hydrochloride (2-amino-5,6-dihydro-6 -methyl-4H-1,3-thiazine hydrochloride), AR-C102222, L-NG-nitroarginine, L-NG-monomethylarginine, L-nitroarginine methyl ester, L-NIO, dexamethasone, estrogen, astaxanthin, and apigenin.

14. The composition of claim 10, wherein the ROCK inhibitor is selected from the group consisting of Y-27632, Ripasudil (K-115), Thiazovivin, Fasudil (HA-1077), GSK429286A, RKI-1447, GSK269962, Netarsudil (AR-13324), Y-39983, ZINC00881524, KD025, Hydroxyfasudil (HA-1100), GSK180736A and AT13148.

15. The composition of claim 10, wherein the estrogen-like substance is selected from the group consisting of estrogen, ethinylestradiol, biochanin A, Glycine Soja extract, isoflavone, Belamcanda Chinensis extract, and Pueraria Mirifica root extract.

16. The composition of any one of claims 1-9, wherein the substance that induces or maintains the expression of COL17A1 in a cell is selected from the group consisting of apocynin, ebselen, celecoxib, apigenin, Y-27632, Ripasudil, 1400W, ethinylestradiol, biochanin A, Necrostatin 1, Apocynum Venetum Leaf/Stem Extract, Morus Alba Leaf Extract, Gymnema Sylvestre Leaf Extract, Green tea extract, Eriobotrya Japonica Leaf Extract, Silybum Marianum Extract, black turmeric extract, Plantago Major Seed Extract, coffee seed extract, Prunus Yedoensis Leaf Extract, Melia Azadirachta Leaf Extract, mandarin orange peel extract, lavender flower extract, Sophora Angustifolia Root Extract, Oryza Sativa Extract, Rosa Canina Fruit Extract, Hamamelis Virginiana Leaf Extract, Eucalyptus Globulus Leaf Extract, Crataegus Cuneata Fruit Extract, and Panax Ginseng Root Extract.

17. The composition of any one of claims 1-9, wherein the substance that inhibits the degradation of COL17A1 in a cell is selected from the group consisting of a neutrophils elastase (ELANE) inhibitor and a matrix metalloproteinase (MMP) inhibitor.

18. The composition of claim 17, the ELANE inhibitor is selected from the group consisting of sivelestat sodium hydrate(Monosodium N-{2-[4-(2,2-dimethylpropanoyloxy)-phenylsulfonylamino]benzoyl}aminoacetate tetrahydrate), ONO-6818 (2-(5-Amino-6-oxo-2-phenylhydropyrimidinyl)-N-[2-(5-tert-butyl-1,3,4-oxadiazol-2-yl)-1-(methylethyl)-2-oxoethylacetamide), α1-antitrypsin (α1-AT), Depelestat (Depelestat), NEI-L1 (sodium trifluoride isopropyl oxopropyl aminocarbonyl pyrrolidine carbonyl methyl propyl aminocarbonyl benzoylaminoacetate), Perilla Ocymoides Leaf Ferment Filtrate, Carum Petroselinum Ferment Filatrate, pepper ferment filtrate, an antibody against ELANE, an siRNA against a gene encoding ELANE, and an antisense oligonucleotide against a gene encoding ELANE.

19. The composition of claim 17, wherein the MMP inhibitor is selected from the group consisting of Marimastat (Marimastat), Batimastat (Batimastat), PD166793, Ro32-3555, WAY170523, UK370106, TIMP1, TIMP2, TIMP3, and TIMP4.

20. The composition of any one of claims 1-9, wherein the genomic stress is either stress of DNA damage due to ultraviolet light, radiation or an anticancer agent, or replication stress associated with cell division.

21. The composition of claim 2, wherein the skin aging is at least one selected from the group consisting of (a) to (f) below:
(a) thinning, weakening, atrophy, or loss of firmness of the epidermis, dermis, and/or adipose tissue;
(b) decrease of the epidermal barrier function or dryness of the skin;
(c) reduction or immaturation of hemidesmosomes in the basement membrane;
(d) loss of fibroblasts in the upper dermis, or crepe wrinkles or fine wrinkles due to dryness of the skin;
(e) wrinkles due to loss of collagen in the dermis; and
(f) spots, senile pigment freckles, or depigmented spots.

22. The composition of claim 2, for use in wrinkle inhibition.

23. The composition of any one of claims 1-22, wherein the composition is a pharmaceutical composition.

24. The composition of any one of claims 1-23, comprising a pharmaceutically acceptable excipient.

25. The composition of any one of claims 1-24, wherein the composition is a liniment.

26. The composition of any one of claims 1-22, wherein the composition is a cosmetic composition.

27. The cosmetic composition of claim 26, comprising the active ingredient at 0.01-15 % by weight.

28. The cosmetic composition of claim 26 or 27, wherein the composition is for use in skin care.

29. The cosmetic composition of any one of claims 26-28, further comprising at least one of an anti-skin aging agent, a skin tone-adjusting agent, an antiinflammatory agent, and a sunscreen agent.

30. A method for screening a substance that is effective in promoting skin wound healing, or preventing or improving a skin ulcer, inhibiting skin aging, regulating cell competition, improving the regenerative ability of epidermal stem cells, preventing or improving a skin disorder caused by an anticancer agent, preventing wrinkles, and/or improving skin roughness, comprising:
(i) contacting a cell with a test substance *in vitro;*
(ii) measuring the expression of COL17A1 in a cell; and
(iii) determining whether the test substance upregulates the expression of COL17A1.

31. A method for assessing skin aging using the expression of COL17A1 in a cell as an indicator.

32. A kit for use in a method for evaluating skin aging, comprising an antibody against COL17A1, a probe against a gene encoding COL17A1, or primers for amplifying the gene.

33. A method for evaluating epidermal stem cells using the expression of COL17A1 in a cell as an indicator.

34. A kit for use in a method for evaluating epidermal stem cells, comprising an antibody against COL17A1, a probe against a gene encoding COL17A1, or primers for amplifying the gene.

35. A method for sorting epidermal stem cells using the expression of COL17A1 in a cell as an indicator.

36. A kit for use in a method for sorting epidermal stem cells, including an antibody against COL17A1, a probe against a gene encoding COL17A1, or primers for amplifying the gene.

37. A method for amplifying epidermal stem cells and/or epidermal cells, comprising measuring the expression level of COL17A1 using an antibody against COL17A1, a probe against a gene encoding COL17A1, or primers for amplifying the gene, and sorting epidermal stem cells with a high expression level of COL17A1.

38. A kit for use in a method of amplifying epidermal stem cells and/or epidermal cells, comprising an antibody against COL17A1, a probe against a gene encoding COL17A1, or primers for amplifying the gene.

39. A functional food or beauty supplement comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

40. The functional food or beauty supplement of claim 38, wherein the substance that induces or maintains the expression of COL17A1 in a cell is selected from the group consisting of an NADPH oxidase inhibitor, a COX inhibitor, an iNOS inhibitor, a ROCK inhibitor, and an estrogen-like substance.

41. The functional food or beauty supplement of claim 39 or 40, wherein the substance that induces or maintains the expression of COL17A1 in a cell is selected from the group consisting of apocynin, ebselen, celecoxib, apigenin, Y-27632, Ripasudil, 1400W, ethinylestradiol, biochanin A, Necrostatin 1, Apocynum Venetum Leaf/Stem Extract, Morus Alba Leaf Extract, Gymnema Sylvestre Leaf Extract, Green tea extract, Eriobotrya Japonica Leaf Extract, Silybum Marianum Extract, black turmeric extract, Plantago Major Seed Extract, coffee seed extract, Prunus Yedoensis Leaf Extract, Melia Azadirachta Leaf Extract, mandarin orange peel extract, lavender flower extract, Sophora Angustifolia Root Extract, Oryza Sativa Extract, Rosa Canina Fruit Extract, Hamamelis Virginiana Leaf Extract, Eucalyptus Globulus Leaf Extract, Crataegus Cuneata Fruit Extract, and Panax Ginseng Root Extract.

42. The functional food or beauty supplement according to any one of claims 39-41, which is intended for oral intake.

43. The functional food or beauty supplement according to any one of claims 39-42, which is in the form of tablet, powder, semi-solid, jelly or liquid.

44. The functional food or beauty supplement according to any one of claims 39-43, further comprising at least one of an anti-skin aging agent, vitamin, collagen, and mineral.

45. A cell composition comprising an isolated cell expressing COL17A1.

46. The cell composition of claim 45, wherein the cell is an epidermal keratinocyte or a mucosal epithelial keratinocyte.

47. The cell composition of claim 45 or 46, which is for use in transplantation.

48. The cell composition according to any one of claims 45-47, which is for use in the treatment of a disease or injury of the skin.

49. The cell composition according to any one of claims 45-48, comprising at least 1 × 10³ cells.

50. The cell composition according to any one of claims 45-49, wherein at least 50% of the cells express COL17A1.

51. The cell composition according to any one of claims 45-50, wherein the cell is a stem cell-derived cell.

52. The cell composition according to any one of claims 45-51, wherein the cell is frozen.

53. The cell composition according to any one of claims 45-52, wherein the cell is contained in a single container.

54. The cell composition according to any one of claims 45-53, wherein the cell is a cell treated with a substance that induces or maintains the expression of COL17A1 in the cell.

55. The cell composition of claim 53, wherein the substance that induces or maintains the expression of COL17A1 in the cell is selected from the group consisting of an NADPH oxidase inhibitor, a COX inhibitor, an iNOS inhibitor, a ROCK inhibitor, and an estrogen-like substance.

56. The cell composition of claim 54 or 55, wherein the substance that induces or maintains the expression of COL17A1 in a cell is selected from the group consisting of apocynin, ebselen, celecoxib, apigenin, Y-27632, Ripasudil, 1400W, ethinylestradiol, biochanin A, Necrostatin 1, Apocynum Venetum Leaf/Stem Extract, Morus Alba Leaf Extract, Gymnema Sylvestre Leaf Extract, Green tea extract, Eriobotrya Japonica Leaf Extract, Silybum Marianum Extract, black turmeric extract, Plantago Major Seed Extract, coffee seed extract, Prunus Yedoensis Leaf Extract, Melia Azadirachta Leaf Extract, mandarin orange peel extract, lavender flower extract, Sophora Angustifolia Root Extract, Oryza Sativa Extract, Rosa Canina Fruit Extract, Hamamelis Virginiana Leaf Extract, Eucalyptus Globulus Leaf Extract, Crataegus Cuneata Fruit Extract, and Panax Ginseng Root Extract.

57. A cell sheet comprising the cell composition according to any one of claims 45-56.

58. A composition for use in regulating cell competition, comprising as an active ingredient a substance selected from the group consisting of a substance that induces or maintains the expression of COL17A1 in a cell, a substance that inhibits the degradation of COL17A1 in a cell, a substance that maintains the self-renewal ability of epidermal stem cells, a substance that inhibits genomic stress or oxidative stress in a cell, and a substance that inhibits DNA damage in a cell.

59. A method for assessing cell competition in keratinocytes by culturing keratinocytes in three dimensions.

60. A kit for use in a method for assessing cell competition in keratinocytes, comprising keratinocytes and a three-dimensional culture device.
